(19) Europäisches Patentamt
European Patent Office
Office européen des brevets

(11) **EP 3 493 835 B1**

(12) **EUROPEAN PATENT SPECIFICATION**

(45) Date of publication and mention
of the grant of the patent:
**16.08.2023 Bulletin 2023/33**

(51) International Patent Classification (IPC):
**A61K 39/00** (2006.01)       **A61K 35/15** (2015.01)
**A61K 35/62** (2006.01)

(21) Application number: **17772758.3**

(52) Cooperative Patent Classification (CPC):
**A61K 39/0003; A61K 35/17; A61K 39/0008;**
A61K 2035/122; A61K 2039/5158; A61K 2039/577

(22) Date of filing: **04.08.2017**

(86) International application number:
**PCT/IB2017/054790**

(87) International publication number:
**WO 2018/025236 (08.02.2018 Gazette 2018/06)**

(54) **METHOD FOR EX VIVO INDUCTION OF SUPPRESSOR LYMPHOCYTES USING A SOLUTION OF PROTEINS FROM HELMINTH PARASITES**

VERFAHREN ZUR EX-VIVO-INDUKTION VON SUPPRESSOR-LYMPHOZYTEN UNTER
VERWENDUNG EINER LÖSUNG VON PROTEINEN AUS HELMINTH-PARASITEN

MÉTHODE D'INDUCTION EX VIVO DE LYMPHOCYTES SUPPRESSEURS À L'AIDE DE
PROTÉINES PROVENANT DE PARASITES D'HELMINTHES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.08.2016 PL 41820216**

(43) Date of publication of application:
**12.06.2019 Bulletin 2019/24**

(73) Proprietor: **Uniwersytet Warszawski
00-927 Warszawa (PL)**

(72) Inventor: **DONSKOW - LYSONIEWSKA, Katarzyna
05-126 Nieporet (PL)**

(74) Representative: **Grzelak, Anna
Sigeon IP, sp z o. o.
ul. Bukowinska 24A m. 65
02-703 Warszawa (PL)**

(56) References cited:
**WO-A1-2013/039872     WO-A1-2013/192215
US-A1- 2005 118 655**

- **MARIELA SEGURA ET AL: "Impairment of dendritic cell function by excretory-secretory products: A potential mechanism for nematode-induced immunosuppression", EUROPEAN JOURNAL OF IMMUNOLOGY, vol. 37, no. 7, 12 June 2007 (2007-06-12), pages 1887-1904, XP055419080, ISSN: 0014-2980, DOI: 10.1002/eji.200636553**
- **A. P. KOHM ET AL: "Cutting Edge: CD4+CD25+ Regulatory T Cells Suppress Antigen-Specific Autoreactive Immune Responses and Central Nervous System Inflammation During Active Experimental Autoimmune Encephalomyelitis", THE JOURNAL OF IMMUNOLOGY, vol. 169, no. 9, 1 November 2002 (2002-11-01), pages 4712-4716, XP055419573, US ISSN: 0022-1767, DOI: 10.4049/jimmunol.169.9.4712 cited in the application**
- **MALOY K J ET AL: "Regulatory T cells in the control of immune pathology", NATURE IMMUNOLOGY,, vol. 2, no. 9, 1 September 2001 (2001-09-01), pages 816-822, XP002261824, ISSN: 1529-2908, DOI: 10.1038/NI0901-816 cited in the application**

- EL-MALKY M ET AL: "Helminth infections: therapeutic potential in autoimmune disorders", 1 November 2011 (2011-11-01), PARASITE IMMUNOLOGY, BLACKWELL SCIENTIFIC PUBLICATIONS, OXFORD, GB, PAGE(S) 589 - 593, XP002696666, ISSN: 0141-9838 [retrieved on 2011-10-13]
- Katarzyna Donskow-Lysoniewska ET AL: "Heligmosomoides polygyrus: EAE remission is correlated with different systemic cytokine profiles provoked by L4 and adult nematodes", EXPERIMENTAL PARASITOLOGY, vol. 132, no. 2, 1 October 2012 (2012-10-01), pages 243-248, XP055701804, US ISSN: 0014-4894, DOI: 10.1016/j.exppara.2012.07.009

- DONSKOW-LYSONIEWSKA KATARZYNA ET AL: "The Effects of Intestinal Nematode L4 Stage on Mouse Experimental Autoimmune Encephalomyelitis", ARCHIVUM IMMUNOLOGIAE ET THERAPIAE EXPERIMENTALIS, BIRKHAEUSER VERLAG AG, CH, vol. 66, no. 3, 3 October 2017 (2017-10-03), pages 231-243, XP036505272, ISSN: 0004-069X, DOI: 10.1007/S00005-017-0489-Z [retrieved on 2017-10-03]

## Description

### *Technical field*

The invention relates to a method for ex *vivo* induction of lymphocytes with a CD8$^+$CD25$^+$ phenotype, in particular with a CD8$^+$CD25$^+$FOXP3$^-$CD103$^-$ phenotype. The use of a solution of somatic and/or excretory-secretory proteins from helminth parasites, and/or functional protein fractions of the above solution in ex *vivo* induction of lymphocytes to obtain induced lymphocytes with a CD8$^+$CD25$^+$FOXP3$^-$ phenotype, preferably with a CD8$^+$CD25$^+$FOXP3$^-$CD103$^-$ phenotype in an individual in need thereof is also described.

### *Background art*

Autoimmune diseases are characterized by a lack of tolerance of the immune system to its own autoantigens. The loss of immunotolerance leads to the emergence of *inter alia* autoreactive T helper (Th) lymphocytes that recognize autoantigens and lead to their destruction in a given target organ.

It is known that the onset of one autoimmune disease predisposes to the development of others. The coexistence of some autoimmune diseases in the families of patients suggests the existence of a common genetic background. However, apart from the genetic determinants, the role of epigenetic factors in the development of autoimmune diseases is increasingly recognized (Lu, 2013). The mechanisms regarded as responsible for pathogenesis include defects in apoptosis of autoreactive T lymphocytes and/or the emergence of new antigens due to atypical posttranslational modifications of histone proteins by the introduction of various additional molecules or functional groups (such as methyl, acetyl, phosphate groups or the ubiquitin protein) to amino acids: lysine and arginine or by the processing of normal cell components that will be recognized as non-self by the immune system (Lu, 2013).

Autoimmune diseases occur mainly in the developed countries in Western Europe, North America and Australia in people aged 20-40 years. To date, no effective treatment of these diseases has been developed.

The therapies used are mainly based on: symptomatic treatment (alleviation of symptoms of the disease), treatment of relapses (relief from their after-effects) and treatment modifying the natural course of the disease (reducing the number of relapses during the year). Standard treatment of autoimmune diseases requires selective qualification of patients for the therapy, what results from the use of drugs, which non-specifically modify or non-specifically suppress the immune system. During treatment, patients require highly specialized assessment as well as exclusion and constant monitoring for oncological hazards. Therefore, antigen-specific therapy is currently the most interesting approach in the treatment of these diseases.

### Inflammatory bowel disease

Inflammatory bowel disease (IBD) includes, *inter alia,* Crohn's disease (CD) and ulcerative colitis (UC). Both diseases are idiopathic chronic diseases with a complex mechanism, in which an interaction between environmental factors and genetic predisposition occurs, and pathogenesis is probably associated with abnormal immune system activation against its own antigens and the antigens of intestinal bacterial flora. The morbidity of IBD is 400 per 100,000 people, including the incidence of ulcerative colitis, which varies between 0.5-24.5 per 100,000 people. In Poland, morbidity is estimated to be approx. 700 cases a year. Probably, however, the incidence of these diseases, and especially ulcerative colitis, is greater due to the lack of the so-called golden standards for diagnostics of diseases; no consistent diagnosis and misclassification of the disease (Lakatos, 2006; Molodecky et al., 2012), despite the currently applicable Montreal classification (Satsangi et al., 2006).

IBD treatment is directed at controlling the inflammatory symptoms. In the treatment, 4 groups of drugs are mainly used:
1. Preparations of 5-aminosalicylic acid (5-ASA): mesalazine and sulfasalazine, used primarily for the treatment of mild to moderate disease relapses. In the form of oral tablets, and rectal infusions and suppositories. 2. Glucocorticosteroids: prednisone - used in moderate ulcerative colitis, and methylprednisolone - administered intravenously to hospitalized patients with a severe relapse of the disease. 3. Immunosuppressant drugs and modulators: (cyclosporine, tacrolimus and a monoclonal antibody - infliximab, azathioprine and methotrexate) - used in the case of resistance or intolerance to glucocorticosteroids. 4. Antibiotics administered orally and rectally.

In one-fourth to one-third of patients with ulcerative colitis, conservative pharmacological therapy is ineffective and complications occur. Long-term treatment causes liver damage and bone marrow disorders. Topical complications lead to ulcer bleeding and colon rupture (Järnerot et al., 1995). Surgical methods used in such a case include resection of a part or the entire large intestine.

However, the most severe consequence of ulcerative colitis is dysplasia of epithelial cells leading to tumor growth, which is 3 times more common in patients with ulcerative colitis than in the rest of the population. Risk factors for colon cancer are long duration of the disease (7 to 10 years) and, in about 0.5-1% of cases per year, endoscopic diagnosis qualifies these lesions for surgical treatment. Approximately 25% of the patients require colectomy. Surgery provides a lasting therapeutic effect, but complete rectal and colon excision with rectum reconstruction significantly lowers the quality of life of the patients. IBD is one of the five most common gastrointestinal diseases, and the total cost of healthcare in the United States is more than $1.7 billion. Surgical treatment and postoperative complications treatment generate the highest cost (Meder et al., 2011).

Multiple sclerosis

Multiple sclerosis (MS, latin: *sclerosis multiplex*) is an idiopathic, chronic autoimmune and neurodegenerative disease with a complex mechanism involving an interaction between environmental factors and genetic predisposition, the pathogenesis of which is probably associated with abnormal activation of the immune system against self-antigens -oligodendrocyte myelin glycoproteins.

The disease occurs mainly in the developed countries of Western Europe, North America and Australia in people aged 20-40 years. The estimated morbidity of MS in the world ranges from 15 to 180 per 100,000 people. It is estimated that in the United States, there are 350,000 to 500,000 people suffering from the disease. In Poland, in the absence of nationwide epidemiological studies, this number is expected to range between 45 and 92 per 100,000 people and tends to increase especially among women (Potemkowski, 2009, Kułakowska et al., 2009).

Currently, in MS treatment, drugs belonging to the following groups are mainly used: glucocorticosteroids (methylprednisolone, Solu-Medrol), which are administered intravenously to hospitalized patients only in severe relapses, significantly affecting the patient's condition; immunomodulatory and immunosuppressive drugs - administered in the relapsing-remitting MS, with at least one relapse per year: Beta-interferons (Avonex, Betaferon, Rebif, Extavia) - with potent anti-inflammatory effect; Glatiramer acetate (Copaxone); Mitoxantrone - an antineoplastic; Cyclophosphamide - anticancer drug; Natalizumab (Tysabri, Antegren) - a monoclonal antibody against the VLA-4 adhesion molecule that reduces leukocyte migration to the interstitial space of the brain (Indeks Leków, 2014; Selmaj, 2005); Fingolimod - blocking the sphingosine 1-phosphate receptors of the type 1, 3, 5 (S1P1, 3, 5) on T and B lymphocytes as well as the CNS neurons, which limits the migration of lymphocytes from lymph nodes to peripheral blood (Indeks Leków, 2014).

Symptomatic treatment involves: 1. Spasticity treatment using such drugs as: baclofen (Baclofen, Lioresal), tizanidine (Sirdalud, Sirdalud MR), tetrazepam (Myolastan, Tetraratio, Myopam), diazepam (Relanium), clonazepam or tolperisone (Mydocalm, Tolperis). 2. Sphincter dysfunction treatment: cholinolytic and cholinomimetic drugs supporting or inhibiting bladder function. 3.

Neuralgia treatment: carbamazepine (Amizepin, Tegretol). 4. Depression treatment: uoxetine, sertraline, tianeptine. 5. Fatigue treatment: amantadine, pemoline, modafinil, hematine, amphetamine or methylphenidate (Indeks Leków, 2014).

The effectiveness of MS treatment mainly refers to the relapsing form of the disease. In the cases of both chronically progressive and secondary progressive disease, there is no effective treatment and the clinical trials of the proposed drugs have failed (Palasik, 2010). In consequence, MS is one of the most serious causes of severe disability in patients and, after traumatic brain injury and childhood cerebral palsy, the third leading cause of disability in young people.

In the state of the art, the dominant role of T-helper (Th) lymphocytes in autoimmune diseases has been demonstrated. In early and fully developed lesions in organ systems Th1 (CD4 and CD8) effector lymphocytes are dominant, which, as a result of the secreted cytokines, activate molecular pathways leading to the induction and maintenance of inflammation in the organ. Th1 lymphocytes with CD4$^+$ phenotype are predominant in early and fully developed autoimmune lesions. The importance of CD8$^+$ lymphocytes in the pathogenesis of diseases is poorly understood and unclear. It is known, however, that while CD4$^+$ lymphocytes play a role in the formation and maintenance of pathological lesions, CD8$^+$ lymphocytes directed against autoantigens are the main effector cells in chronic disease and they control polarization of Th1 lymphocytes (Gudjonsson et al., 2004; Friese and Fugger, 2005). As it was demonstrated, *inter alia* in psoriasis, the development of lesions is associated in the early stage with the infiltration and activation of CD4$^+$ lymphocytes, while in the later phase, during spontaneous recurrence of lesions, CD8$^+$ lymphocytes dominate (Gudjonsson et al., 2004). It has been suggested, but not demonstrated, that this may be due to the appearance of subpopulations of CD8$^+$ lymphocytes with suppressor properties, which contribute to the blocking of activity/activation of autoreactive lymphocytes and inhibition of inflammatory response (Stock et al., 2004) and the mechanism may be similar to that observed during thermal injury, where CD8$^+$ lymphocytes play a major role in the inhibition of the inflammatory response (Gryglewski et al., 2006). The role of suppressor CD8$^+$ cells in reversing autoimmune lesions is confirmed *inter alia* by the works on the use of UV-B radiation in the treatment of psoriasis (Piskin et al., 2004).

In the state of the art, helminthic therapy - that is the use of helminth parasites for the treatment of autoimmune diseases, is known. To date, the most effective biological therapy for ulcerative colitis is infection with life nematodes *Trichuris suis* and *Necator americanus,* on which clinical trials have begun (Ruyssers et al., 2008; Fleming et al., 2011). The therapy requires constant presence of the parasite within the patient's body, which for many patients is unacceptable; causes a mental discomfort and a number of common side effects, such as tissue damage by migrating nematode larvae, weakening in physical and intellectual condition. Until now, the studies on the therapeutic application of recombinant parasite proteins for potential use in the treatment of autoimmune diseases have not provided satisfactory results.

In mice with experimental autoimmune encephalomyelitis (EAE), used as the basic model in the studies on multiple sclerosis based on autoimmunological, histopathological, genetic and clinical similarities, infected by an intestinal nematode *Heligmosomoides polygyrus,* remission of the symptoms of the disease takes place from the second day after the infection (Donskow-Lysoniewska et al., 2012).

In the paper: Liu, Y., Xu, A. P., Horwitz, D. A., & Zheng, S. G. (2012). Cd8+ foxp3-cd103+ Regulatory T Cells Generated Ex Vivo with Tgf-$\beta$ Suppress Autoimmunity Through Il-10-dependent Mechanism. Arthritis & Rheumatism, 64, S105 and Liu, Y., Lan, Q., Lu, L., Chen, M., Xia, Z., Ma, J., ... & Brand, D. (2013). Phenotypic and functional characteristic of a newly identified CD8+ Foxp3- CD103+ regulatory T cells. Journal of molecular cell biology, 6(1), 81-92, $CD8^+FOXP3^-CD103^+$ cells induced by transforming growth factor TGF-$\beta$, were described and indicated as inhibiting inflammation in the mouse model of EAE and colitis.

Document JP2012008133A relates to the composition of parasites and the method of using thereof in prevention and treatment of the state of disease, being the result of a modified immunological response.

Document RU2437933 relates to the method of enhancing human lymphocytes $CD4^+CD25^+$ $FOXP3^+$.

In the patent US6521232 B1 the use of isolated proteins containing amino acid sequences of helminth proteins with immunomodulatory properties as therapeutic factors for the treatment of immunological diseases was described.

Document WO2013/039872 A1 relates to helminth-based therapeutic compositions and methods for treating inflammatory bowel disease, including Crohn's disease and ulcerative colitis.

Document US2005/118655 relates to a method of screening a heminthic parasite preparation that affects a regulatory T cell function and a method of treating a disease by altering a regulatory T cell activity through the administration of a parasite preparation.

Document WO2013/192215 A1 describes the possible use of regulatory T lymphocytes in the treatment of autoimmune and inflammatory conditions.

Document WO2006/081372 A2 relates to the use of a compound obtained from helminths which do not colonize humans, for administration to patients to simulate parasitic infection and therefore to eliminate or relieve inappropriate immunological reaction in patients with asthma or allergy.

In the European patent EP 1 530 972 the use of helminth parasites to modify the activity of regulatory T-cells, characterized by a high level of FOXP3 transcription ($FOXP3^+$) was described and indicated.

### *Disclosure of invention*

The invention is set out in the appended set of claims. The embodiments and/or examples of the following description which are not covered by the appended claims are considered as not being part of the present invention. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The invention relates to a method for ex *vivo* induction of suppressor lymphocytes with a $CD8^+CD25^+$ phenotype, to obtain induced lymphocytes with a $CD8^+CD25^+FOXP3^-$ phenotype, in particular with a $CD8^+CD25^+FOXP3^-CD103^-$ phenotype.

The essence of the present invention is based on an unexpected finding that in mice with experimental autoimmune encephalomyelitis (EAE), infected by an intestinal nematode *Heligmosomoides polygyrus,* EAE remission is correlated with the increase in the percentage of lymphocytes with the $CD8^+CD25^+FOXP3^-$ phenotype, and particularly $CD8^+CD25^+FOXP3^-CD103^-$, including CD25hi (with a high CD25 expression) in blood, cerebrospinal fluid and brain. Most lymphocytes with this phenotype are observed during the 6th day of the infection, when nematode larvae in the L4 stage are present in the muscles of the small intestine. On this day a complete lack of EAE symptoms is observed. By EAE symptoms it is meant neurological symptoms, such as: tail flaccidity, front and/or hind limb paresis, paralysis of the tail and/or paralysis of front and/or hind limbs.

Further studies showed that the lymphocytes with the $CD8^+CD25^+FOXP3^-$ phenotype, isolated from cerebrospinal fluid and blood of mice with EAE infected with a nematode, *in vitro* inhibit proliferation of MOG-specific lymphocytes, while not affecting the proliferation activity of non-specific lymphocytes, wherein lymphocytes with the $CD8^+CD25^+$ phenotype isolated form mice with EAE uninfected by nematodes, as well as from mice only infected by nematodes do not show as high inhibitory effect on the proliferation of MOG-specific lymphocytes.

During *in vivo* studies, it was unexpectedly found that it is possible to carry out adaptive transfer (by intravenous injection) of lymphocytes with the $CD8^+CD25^+FOXP3^-$ phenotype, and in particular $CD8^+CD25^+FOXP3^-CD103^-$, isolated from diseased mice (with induced EAE) and infected by an intestinal nematode *H. polygyrus* to diseased mice with EAE, which causes inhibition of the symptoms of the disease starting from the 2nd day after injection. Unexpectedly, therapeutic effect was observed after a transfer of a very small dose of 3000 lymphocytes CD8$^+$CD25$^+$FOXP3$^-$. The effect of application of that dose of lymphocytes CD8$^+$CD25$^+$FOXP3$^-$lasted until the end of the experiment - for 28 days. The mechanism was indicated *in vivo:* CD8$^+$CD25$^+$FOXP3$^-$ cells pass through the brain-blood barrier, infiltrate the cerebrospinal fluid and brain where they intensively proliferate and limit the proliferation of MOG-specific lymphocytes and production of proinflammatory cytokines by these lymphocytes, which leads to inhibition of demyelination.

As it was unexpectedly found, therapeutic effect of the transfer of CD8$^+$CD25$^+$FOXP3$^-$lymphocytes, preferably CD8$^+$CD25$^+$FOXP3$^-$CD103$^-$ isolated from animals with EAE, infected with a nematode, was much stronger than that caused by an injection of the same or even several times larger dose of CD4$^+$CD25$^+$FOXP3$^+$ lymphocytes, isolated from the same animals and observed by others (Kohm et al., 2002). This is a particularly promising result as the regulatory lymphocytes CD4$^+$CD25$^+$FOXP3$^+$ are currently regarded as the lymphocytes with the highest immunosuppressive potential, which determine the inhibition of inflammatory response in allergies and autoimmune diseases, and which functionality may affect the course of the disease (Putzki and Hartung 2009; Maloy and Powrie, 2001).

The latest *in vitro* studies carried out by the present inventors demonstrated that whole blood as well as isolated peripheral blood mononuclear cells (PBMC) of diseased mice with induced EAE, subjected to the activity of a solution of somatic proteins from L4 stage larvae of a nematode *H. polygyrus* differentiate into lymphocytes with a CD8$^+$CD25$^+$ phenotype, the percentage of which increases from approximately 5% to 22% of the total population of lymphocytes in the blood of mice, whereas the induced lymphocytes do not show expression of the surface marker CD103 nor the transcription factor FOXP3, thus the obtained suppressor CD8Ts lymphocytes are CD8$^+$CD25$^+$FOXP3$^-$CD103$^-$.

The therapeutic effect of CD8$^+$CD25$^+$ lymphocytes activated by nematode antigens was also demonstrated in the treatment of ulcerative colitis induced by dextran sulfate sodium (DSS) in mice. Remission of colitis symptoms has been documented (starting from) the 2nd day after the injection of 3000 CD8$^+$CD25$^+$ lymphocytes. This result indicates a therapeutic potential of CD8$^+$CD25$^+$ lymphocytes induced in this way in other autoimmune diseases.

In this way a new potential therapeutic method for treatment and/or alleviation of symptoms of autoimmune diseases was found.

The invention relates to a method for ex *vivo* induction of lymphocytes with a CD8$^+$CD25$^+$ phenotype, wherein isolated blood cells of an individual, are ex *vivo* contacted with a solution of somatic proteins from a helminth parasite, wherein the solution of somatic proteins is obtained by collecting developmental stage of a helminth L4 larvae stage and shredding in order to obtain a complete tissue homogenate, and wherein helminth parasite is a nematode *Heligmosomoides polygyrus;* wherein the contacting is carried out under conditions and on a medium enabling proliferation of lymphocytes, wherein the induced lymphocytes are then isolated, wherein the obtained isolated lymphocytes with the CD8$^+$CD25$^+$ phenotype do not express the transcription factor FOXP3 and are CD103$^-$; wherein the L4 stage of development of *Heligmosomoides polygyrus* is obtained by infection of a non-human animal with an invasive form, wherein preferably the infection is with the L3 stage of development of *Heligmosomoides polygyrus;* cultivation of the non-human animal for the time needed for the larva to transform into the next stage L4, in which it colonizes the small intestine; isolation of the small intestine of the non-human animal and release of the developed L4 stage into the medium and its separation from the medium; and wherein isolated blood cells of an individual are from individual exhibiting presence of autoreactive T lymphocytes.

In the preferred method of invention for ex *vivo* induction of lymphocytes with a CD8$^+$CD25$^+$ phenotype the isolated blood cells of an individual come from a human; wherein preferably isolated blood cells are whole blood or peripheral blood mononuclear cells.

In the preferred method of invention for ex *vivo* induction of lymphocytes with a CD8$^+$CD25$^+$ phenotype the proliferation of the lymphocytes is carried out in a culturing medium enabling proliferation of lymphocytes containing 10% inactivated human serum, for 24-120 hours under the conditions of 37°C, 5%$CO_2$ and 95% humidity.

In the preferred method of invention for ex *vivo* induction of lymphocytes with a CD8$^+$CD25$^+$ phenotype the obtained induced lymphocytes with the phenotype CD8$^+$CD25$^+$ FOXP3$^-$CD103$^-$, are then isolated based on the expression of markers CD8, CD25, CD103, preferably also CD3, using a cell sorter, using appropriately labeled monoclonal antibodies against the particular markers, preferably anti-human antibodies, and the FOXP3$^-$ factor is also identified.

The description teaches a method for ex *vivo* induction of lymphocytes with a CD8$^+$CD25$^+$ phenotype, wherein isolated blood cells of an individual are ex *vivo* contacted with a solution of somatic proteins and/or excretory-secretory proteins from a helminth parasite, wherein the solution of somatic proteins is obtained by collecting at least one developmental stage of a helminth, preferably a nematode, selected from eggs, L1, L2, L3, L4 larvae, pre-adult stage L5, adult form, and shredding in order to obtain a complete tissue homogenate and wherein the solution of excretory-secretory proteins is obtained by collecting a helminth, preferably a nematode, of at least one developmental stage, selected from larvae L4, pre-adult stage L5, adult form, and incubating it in a medium and obtaining a solution of excretory-secretory proteins in the form of a solution of products secreted by this form to the medium, wherein the contacting is carried out under conditions and on a medium enabling proliferation of lymphocytes, wherein the induced lymphocytes are then isolated, and wherein the obtained isolated lymphocytes with the CD8$^+$CD25$^+$ phenotype do not express the transcription factor FOXP3, and further are more preferably CD103$^-$.

In the described method for ex *vivo* induction of lymphocytes with the CD8$^+$CD25$^+$ phenotype, the helminth parasite is a nematode *Heligmosomoides polygyrus.*

In the described method for ex *vivo* induction of lymphocytes with the CD8$^+$CD25$^+$ phenotype, the isolated blood cells of an individual are ex *vivo* contacted with a solution of somatic proteins and/or excretory-secretory proteins from the L4 developmental stage of *Heligmosomoides polygyrus,* preferably the isolated blood cells of an individual are ex *vivo* contacted with a solution of somatic proteins from the L4 developmental stage of *Heligmosomoides polygyrus.*

In the described method for ex *vivo* induction of lymphocytes with the CD8$^+$CD25$^+$ phenotype, the L4 developmental stage of *Heligmosomoides polygyrus* is obtained by infecting a non-human animal with an invasive form, preferably in the L3 stage, cultivation of a non-human animal for the time needed for the larva to transform into the next stage, L4, in which it colonizes the small intestine, isolation of the small intestine of the non-human animal and release of the developed L4 stage into the medium and its separation from the medium.

In the described method for ex *vivo* induction of lymphocytes with the CD8$^+$CD25$^+$ phenotype, the isolated blood cells of an individual come from a human.

In the described method for ex *vivo* induction of lymphocytes with the CD8$^+$CD25$^+$ phenotype, the isolated blood cells are whole blood cells or peripheral blood mononuclear cells.

In the described method for ex *vivo* induction of lymphocytes with the CD8$^+$CD25$^+$ phenotype, proliferation of the lymphocytes is carried out in the complete RPMI-1640 containing: 10% inactivated human serum, 20 mM L-glutamine, 10 μg/ml gentamicin and 1 M HEPES for 24-120 hours, under the conditions of 37°C, 5%CO$_2$ and 95% humidity.

In the described method for ex *vivo* induction of lymphocytes with the CD8$^+$CD25$^+$ phenotype, the induced lymphocytes with the CD8$^+$CD25$^+$ FOXP3$^-$ phenotype, preferably CD8$^+$CD25$^+$ FOXP3$^-$CD103$^-$, are then isolated based on the expression of markers CD8, CD25, CD103, preferably also CD3, in a cell sorter using appropriately labeled monoclonal antibodies against the particular markers, preferably anti-human antibodies, and the FOXP3$^-$ factor is also identified.

In the described method for ex *vivo* induction of lymphocytes with the CD8$^+$CD25$^+$ phenotype, the individual from which isolated blood cells are obtained is a human with autoreactive T lymphocytes, in particular as a result of autoimmune diseases.

In the described method for ex *vivo* induction of lymphocytes with the CD8$^+$CD25$^+$ phenotype, the presence of autoreactive T lymphocytes in a human is a result of an autoimmune disease, selected from non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis.

In the described method for ex *vivo* induction of lymphocytes with the CD8$^+$CD25$^+$ phenotype, the isolated blood cells are obtained from a human with an autoimmune disease, which is ulcerative colitis and/or multiple sclerosis.

In the described method for ex *vivo* induction of lymphocytes with the CD8$^+$CD25$^+$ phenotype, the human from which the isolated blood cells are obtained is administered at least one or more therapeutic agent used in the symptomatic treatment of the disease and/or immunomodulating the disease related to the presence of autoreactive T lymphocytes.

In the described method for ex *vivo* induction of lymphocytes with the CD8$^+$CD25$^+$ phenotype, the human from whom the isolated cells are obtained, is or will be administered CD4$^+$CD25$^+$FOXP3$^+$ lymphocytes, also induced ex *vivo*.

In the described method for ex *vivo* induction of lymphocytes with the CD8$^+$CD25$^+$ phenotype, the somatic protein solution and/or the solution of excretory-secretory proteins from a helminth parasite is a solution of a functional somatic protein and/or an excretory-secretory protein, and/or a recombinant somatic protein and/or an excretory-secretory protein or a protein with amino acid sequence identical in at least 70% with the abovementioned proteins.

It is also described but not covered by the subject-matter of the claims a use of a solution of somatic proteins and/or excretory-secretory proteins of a helminth parasite in a method for ex *vivo* induction of lymphocytes with a CD8$^+$CD25$^+$ phenotype in isolated blood cells of an individual, wherein the blood cells of the individual are *ex vivo* contacted with a solution of somatic proteins and/or excretory-secretory proteins from a helminth parasite, wherein the solution of somatic proteins is obtained by collecting at least one developmental stage of a helminth, preferably a nematode selected from L4 larva, pre-adult stage L5, adult form, and shredding in order to obtain a complete tissue homogenate and wherein the solution of excretory-secretory proteins is obtained by collecting a helminth, preferably a nematode of at least one developmental stage, selected from larvae L4, pre-adult stage L5, adult form, and incubating it in a medium and obtaining a solution of excretory-secretory proteins in the form of a solution of products secreted by this form to the medium, where the contacting is carried out under conditions and on a medium enabling proliferation of lymphocytes, where the induced lymphocytes are then isolated, and wherein the obtained induced lymphocytes with the CD8$^+$CD25$^+$ phenotype do not express the transcription factor FOXP3, and further are more preferably CD103$^-$,

wherein preferably, the contacting is carried out under conditions and on a medium enabling proliferation of lymphocytes, and the induced lymphocytes are then isolated.

In the described use of the solution of somatic and/or excretory-secretory proteins from a helminth parasite, the helminth parasite is the nematode *Heligmosomoides polygyrus,* wherein more preferably the isolated blood cells of an individual are ex *vivo* contacted with a solution of somatic proteins and/or excretory-secretory proteins from the L4 developmental stage of *Heligmosomoides polygyrus,* more preferably the isolated blood cells of an individual are *ex vivo* contacted with a solution of somatic proteins.

In the described use of the solution of somatic and/or excretory-secretory proteins from a helminth parasite, the L4 developmental stage of *Heligmosomoides polygyrus* is obtained by infecting with an invasive form, preferably in the L3 stage, a non-human animal, cultivation of the non-human animal for the time needed for the larva to transform into the next stage, L4, in which it colonizes the small intestine; isolation of the small intestine of the non-human animal and release of the developed L4 stage into the medium and its separation from the medium.

In the described use of the solution of somatic and/or excretory-secretory proteins from a helminth parasite, the isolated blood cells of an individual come from a human, preferably with the presence of autoreactive T lymphocytes, in particular as a result of autoimmune diseases.

In the described use of the solution of somatic and/or excretory-secretory proteins from a helminth parasite, the presence of autoreactive T lymphocytes in a human is the result of an autoimmune disease selected from non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis, preferably the autoimmune disease is ulcerative colitis and/or multiple sclerosis.

It is also described but not covered by the subject-matter of the claims induced, isolated human lymphocytes with a CD8$^+$CD25$^+$FOXP3$^-$CD103$^-$ phenotype for use in alleviating the symptoms and treatment of diseases selected from non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis.

The described induced, isolated human lymphocytes with the CD8$^+$CD25$^+$FOXP3$^-$CD103$^-$phenotype are used for alleviating the symptoms and treatment of ulcerative colitis or amyotrophic lateral sclerosis.

The described induced, isolated human lymphocytes with the CD8$^+$CD25$^+$FOXP3$^-$CD103$^-$phenotype for use in alleviating the symptoms and treatment of diseases are transferred to the same individual from the blood of which they were induced.

The described induced, isolated human lymphocytes with the CD8$^+$CD25$^+$FOXP3$^-$CD103$^-$phenotype for use in alleviating the symptoms and treatment of diseases were produced by a method for ex *vivo* induction of lymphocytes with a CD8$^+$CD25$^+$ phenotype, in which the isolated blood cells of an individual are ex *vivo* contacted with the solution of somatic and/or excretory-secretory proteins from a helminth parasite.

It is also described but not covered by the subject-matter of the claims induced human lymphocytes with a CD8$^+$CD25$^+$FOXP3$^-$CD103$^-$ phenotype for use in alleviating the symptoms and treatment of diseases related to the presence of autoreactive T lymphocytes, resulting from autoimmune diseases in a human individual, wherein the autoimmune disease is selected from non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis, wherein the induced CD8$^+$CD25$^+$FOXP3$^-$CD103$^-$ lymphocytes are produced by a method for ex *vivo* induction of lymphocytes with a CD8$^+$CD25$^+$ phenotype, wherein the blood cells isolated from a human individual, showing at least one of the above diseases, are ex *vivo* contacted with a solution of somatic and/or excretory-secretory proteins from a helminth parasite, wherein the solution of somatic proteins is obtained by collecting at least one developmental stage of a helminth, preferably a nematode, selected from eggs, L1, L2, L3, L4 larvae, pre-adult stage L5, adult form, and shredding in order to obtain a complete tissue homogenate, and wherein the solution of excretory-secretory proteins is obtained by collecting a helminths, preferably a nematode of at least one developmental stage, selected from larvae L4, pre-adult stage L5, adult form, and incubating it in a medium and obtaining a solution of excretory-secretory proteins in the form of a solution of products secreted by this form to the medium, wherein the contacting is carried out under conditions and on a medium enabling proliferation of lymphocytes, wherein the induced lymphocytes are isolated based on the expression of CD8, CD25, CD103, preferably also CD3, using a cell sorter and appropriately labeled monoclonal antibodies against particular markers, preferably anti-human antibodies.

The described induced lymphocytes with the CD8$^+$CD25$^+$FOXP3$^-$CD103$^-$ phenotype for use in alleviating the symptoms and treatment of diseases related to the presence of autoreactive T lymphocytes resulting from autoimmune diseases in a human individual, are produced by the method for *ex vivo* induction, in which the helminth parasite is the nematode *Heligmosomoides polygyrus,* wherein preferably the isolated blood cells of an individual are *ex vivo* contacted with the solution of somatic and/or excretory-secretory proteins from the L4 developmental stage of *Heligmosomoides polygyrus,* more preferably the isolated blood cells of an individual are *ex vivo* contacted with a solution of somatic proteins from the L4 developmental stage of *Heligmosomoides polygyrus.*

The described induced lymphocytes with the CD8$^+$CD25$^+$FOXP3$^-$CD103$^-$ phenotype for use in alleviating the symptoms and treatment of diseases related to the presence of autoreactive T lymphocytes resulting from autoimmune diseases in a human individual, are transferred to the same individual from the blood of which they were induced.

Moreover, it is also described but not covered by the subject-matter of the claims a use of a solution of somatic and/or excretory-secretory proteins from a helminth parasite and/or a functional somatic and/or functional excretory-secretory protein solution of a helminth parasite, and/or of a recombinant protein with amino acid sequence identical in at least 70% with a somatic protein and/or an excretory-secretory protein of a helminth, retaining the activity of the protein of the abovementioned solution for *ex-vivo* induction of human lymphocytes with the CD8$^+$CD25$^+$FOXP3$^-$103CD$^-$ phenotype, in an isolated blood of an individual in need thereof, wherein the individual is diagnosed as suffering from or suspected to be suffering from an autoimmune disease related to the presence of autoreactive T lymphocytes, in particular an autoimmune disease, selected from non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis.

It is also described but not covered by the subject-matter of the claims a method for treatment and/or prevention of diseases related to the presence of autoreactive T lymphocytes in autoimmune diseases in humans, selected from non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis, wherein an individual is administered with lymphocytes with the CD8$^+$CD25$^+$FOXP3$^-$CD103$^-$ phenotype are induced by an *ex vivo* method for inducing lymphocytes with a CD8$^+$CD25$^+$ phenotype, wherein the isolated blood cells of an individual are *ex vivo* contacted with a solution of somatic proteins and/or excretory-secretory proteins from a helminth parasite.

It is also described but not covered by the subject-matter of the claims a method for inducing lymphocytes with a CD8$^+$CD25$^+$ phenotype, in which isolated blood cells of an individual are *ex vivo* contacted with a solution of somatic proteins and/or excretory-secretory proteins from a helminth parasite and/or functional protein fractions of the abovementioned solution and/or recombinant proteins with amino acid sequence identical in at least 70% with the proteins in the abovementioned solution; wherein the induced lymphocytes with the CD8$^+$CD25$^+$ phenotype do not express the transcription factor FOXP3, and in the present solution they are referred to as suppressor lymphocytes with the phenotype CD8$^+$CD25$^+$FOXP3$^-$.

Sequence identity is considered to be a measure to which two amino acid sequences are consistent. The identity may be determined by a specialist in the field using the known proteomic methods such as, *inter alia,* mass spectroscopy and/or bioinformatic methods known in the art.

The term "recombinant protein with amino acid sequence identical at a specific percentage to a given protein from the solution of somatic and/or excretory-secretory proteins from a helminth parasite, more preferably with a solution of proteins from an intestinal nematode *H. polygyrus"* is to be understood as a recombinant protein with the amino acid sequence identical in at least 70%, more preferably, in at least 80%, more preferably 85%, more preferably 90%, the most preferably 95% or higher with the amino acid sequence encoding such a recombinant protein.

The solution of somatic and excretory-secretory proteins from a helminth parasite can be obtained by methods known in the art (Valanparambil et al., 2014; Johnston et al., 2015), including, *inter alia* preferably for the solution of somatic proteins by the method described in the Example 5 below.

The solution of somatic proteins of a helminth parasite as used herein is a solution of all structural proteins of the parasite - a complete tissue homogenate.

The solution of excretory-secretory proteins of a helminth parasite as used herein is a solution of all secretions and excretions of the parasite obtained from an *in vitro* culture of the parasite.

The term "protein solution" according to the present invention is synonymous with the term "solution of somatic and/or excretory-secretory proteins".

Functional protein fractions according to the present invention are peptide and protein fractions of a solution of somatic proteins and/or a solution of excretory-secretory proteins with a potential for inducing suppressor CD8$^+$CD25$^+$FOXP3$^-$ lymphocytes, preferably CD8$^+$CD25$^+$FOXP3$^-$CD103$^-$and prepared by known methods and procedures for the separation of peptides and proteins, including electrophoretic methods and chromatographic methods, e.g., HPLC, FPLC, GPC.

The isolated blood cells used in the method according to the invention can be isolated from the body of an individual by methods known in the art., for example by a vacuum method from an ulnar vein into Vacuette tubes with heparin sodium in the amount of 14 I.U./ml or EDTA in the amount of 1.5-2.0 mg/ml of blood.

An individual, in the meaning of the present invention, may be an animal, preferably a mammal, more preferably a human.

In the described method for *ex vivo* induction of lymphocytes with the CD8$^+$CD25$^+$FOXP3$^-$phenotype, preferably with the CD8$^+$CD25$^+$FOXP3$^-$CD103$^-$ phenotype the isolated blood cells of an individual are contacted with:

- a solution of somatic and excretory-secretory proteins from a helminth parasite and/or functional protein fractions of the abovementioned solution, wherein the solution or functional protein fractions contain at least one somatic protein and/or at least one excretory-secretory protein from a helminth parasite, or

- recombinant proteins with amino acid sequence identical in at least 70% with one or more somatic proteins and/or excretory-secretory proteins of a helminth parasite.

Induction, in the meaning of the present invention, is to be understood as a statistically significant increase in the percentage of the CD8$^+$CD25$^+$FOXP3$^-$ lymphocyte population, preferably an increase in the percentage of the CD8$^+$CD25$^+$FOXP3$^-$CD103$^-$ lymphocytes in the population of all blood leukocytes and/or the population of CD8$^+$CD25$^+$ lymphocytes, and/or an increase in the CD25 expression (receptor density) on the CD8$^+$CD25$^+$ lymphocytes and acquisition of immunosuppressive properties providing an *in vivo* therapeutic effect by the CD8$^+$CD25$^+$ lymphocytes, which is obtained by *ex vivo* contacting blood cells with a solution of somatic proteins and/or a solution of excretory-secretory proteins from a helminth parasite and/or functional protein fractions of the abovementioned solution, and/or recombinant proteins with amino acid sequence identical in at least 70% with the proteins in the abovementioned solution, retaining the activity of proteins of the solution.

A skilled person will be able to determine the expression of the transcription factor FOXP3 using methods known in the art, *inter alia,* by flow cytometry, immunohistochemistry, immunoblotting.

The method, wherein recombinant proteins with amino acid sequence identical in at least 70% with the proteins in the abovementioned solution of proteins of a helminth parasite are used for *ex vivo* induction of lymphocytes with a CD8$^+$CD25$^+$FOXP3$^-$ phenotype is also described.

In a preferred described method for *ex vivo* induction of lymphocytes with the CD8$^+$CD25$^+$FOXP3$^-$phenotype, the helminth parasites are larvae of the nematode *Heligmosomoides polygyrus,* all the developmental stages - eggs, L1, L2, L3, L4 larvae, pre-adult stage L5, preferably L4 and adult forms of the nematode *Heligmosomoides polygyrus.*

The description teaches lymphocytes with a CD8$^+$CD25$^+$FOXP3$^-$ phenotype, induced by the method according to the invention for use in the treatment and prevention of autoimmune diseases related to the presence of autoreactive T lymphocytes, particularly autoimmune diseases in an individual.

The described disease is selected from the following group: autoimmune disease such as non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as it can be selected from Parkinson's disease, Alzheimer's disease and amyotrophic lateral sclerosis, for which the presence of autoreactive T lymphocytes has also been shown (Lewis et al., 2012). It is particularly preferred when the disease is multiple sclerosis and/or ulcerative colitis.

In described lymphocytes for use as disclosed, the individual is also administered at least one or more therapeutic agents used in the symptomatic and/or immunomodulating treatment of the disease related to the presence of autoreactive T lymphocytes such as, e.g., cholinolytic drugs, antidepressants, antibiotics, and immunomodulators such as beta-interferons, acting through increasing the number of regulatory CD4$^+$CD25$^+$FOXP3$^+$ lymphocytes, wherein the administration is separated in time or concurrent.

In described embodiment of lymphocytes for described use, the individual is also administered CD4$^+$CD25$^+$FOXP3$^+$ lymphocytes, previously induced *ex vivo.*

Attempts to *ex vivo* induce CD4$^+$CD25$^+$FOXP3$^+$ lymphocytes and to use them in the treatment of autoimmune diseases are known in the art, as described above. These lymphocytes, induced with a solution of somatic proteins and/or a solution of excretory-secretory proteins from helminth parasites, can be used as an additional supplement in the use of described lymphocytes, wherein, as it was demonstrated, the therapeutic success is determined by the use of CD8$^+$CD25$^+$FOXP3$^-$lymphocytes, more preferably CD8$^+$CD25$^+$FOXP3$^-$CD103$^-$.

It is also described but not covered by the subject-matter of the claims a solution of somatic proteins and/or a solution of excretory-secretory proteins from helminth parasites and/or functional protein fractions of the abovementioned solution, and/or recombinant proteins with amino acid sequence identical in at least 70% with the proteins in the abovementioned solution, retaining the activity of proteins of the solution for use in methods for inducing lymphocytes with a CD8$^+$CD25$^+$FOXP3$^-$phenotype (not expressing the transcriptional factor FOXP3), in blood, cerebrospinal fluid and/or brain of an individual, wherein the individual is diagnosed as suffering from or suspected to be suffering from an autoimmune disease related to the presence of autoreactive T lymphocytes, in particular an autoimmune disease.

In described solution of somatic and excretory-secretory proteins and/or functional protein fractions and/or recombinant proteins for described use contain at least one somatic and/or excretory-secretory protein of a helminth parasite, or proteins with amino acid sequence identical in at least 70% with the abovementioned proteins.

In described solution of somatic and excretory-secretory proteins and/or functional protein fractions and/or recombinant proteins for described use the helminth parasites are larvae of the nematode *Heligmosomoides polygyrus,* including all the developmental stages - eggs, L1, L2, L3, L4 larvae, pre-adult stage L5, preferably L4 and adult forms of the nematode *Heligmosomoides polygyrus.*

In described solution of somatic and excretory-secretory proteins and/or functional protein fractions and/or recombinant proteins for described use the disease is selected from the following group: autoimmune disease such as non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease and amyotrophic lateral sclerosis, for which the presence of autoreactive T lymphocytes has also been shown (Lewis et al., 2012).

Preferably, the disease is ulcerative colitis and/or multiple sclerosis.

In a preferred embodiment of the solution of somatic and excretory-secretory proteins and/or functional protein fractions and/or recombinant proteins for described use the individual is also administered at least one or more therapeutic agents used in the symptomatic and/or immunomodulating treatment of the disease related to the presence of autoreactive T lymphocytes such as, e.g., cholinolytic drugs, antidepressants, antibiotics, and immunomodulators such as beta-interferons, acting through increasing the number of regulatory CD4$^+$CD25$^+$FOXP3$^+$ lymphocytes, wherein the administration is separated in time or concurrent.

In a preferred described embodiment of the solution of somatic and excretory-secretory proteins and/or functional protein fractions and/or recombinant proteins for described use the invention the individual is also administered CD4$^+$CD25$^+$FOXP3$^+$ lymphocytes, previously induced *ex vivo.*

The description teaches method for *ex vivo* induction of CD8$^+$CD25$^+$FOXP3$^-$ suppressor lymphocytes with immunomodulatory, anti-inflammatory properties. The description teaches an inducing factor, which is a solution of somatic proteins and/or a solution of excretory-secretory proteins of a helminth parasite, preferably a solution of somatic proteins of *H. polygyrus* L4 stage larvae.

There are described lymphocytes induced ex *vivo,* for therapeutic use in the therapeutic treatment of autoimmune diseases. In therapeutic use, the patient's peripheral blood leukocytes are cultured *in vitro* in the presence of a solution of somatic proteins and/or a solution of excretory-secretory proteins, e.g. a solution of somatic proteins of nematode L4 stage larvae, obtained according to standardized procedures. The solution of somatic proteins of a parasite is obtained by a method known to a person skilled in the art, preferably according to the procedure described in Example 5.

During nematode invasion, mechanisms of inflammatory reaction inhibition, similar to those that occur at the time of hookworm *Necator americanus* infection in humans, are reported. It turns out that somatic and excretory-secretory proteins of helminth parasites, and especially nematodes in the stages parasitizing the host organism, e.g. L4 larvae and adult form, are highly homologous (McSorley et al., 2013), to the extent that enables the use of a helminth parasitizing one species of animal to produce a solution of parasite somatic proteins therefrom, which may be used for ex *vivo* induction of lymphocytes in another species. Hence, it is possible to produce a solution of somatic proteins and/or a solution of excretory-secretory proteins of helminths, particularly nematodes, preferably *H. polygyrus,* particularly *H. polygyrus* L4 stage larvae, obtained from an animal that is not human, preferably mice, and the use of such parasitic somatic and excretory-secretory proteins for the *ex vivo* induction of human suppressor CD8$^+$CD25$^+$ lymphocytes in order to increase the percentage of CD8$^+$CD25$^+$FOXP3$^-$ lymphocytes, preferably CD8$^+$CD25$^+$FOXP3$^-$CD103$^-$ with immunomodulatory anti-inflammatory properties.

The adult form, eggs and larvae at subsequent stages of development, L1, L2, L3, L4 and the pre-adult stage L5 can be shredded, for example, by sonication, which will lead to obtaining of a solution of somatic proteins from the given stage of development. The strongest reaction of the immune system occurs in response to the proteins of the adult form and the so-called tissue larvae in the L4 stage.

For example, the solution of nematode somatic proteins is obtained by infecting a given species of a non-human animal, preferably mouse, rat, rabbit, guinea pig, pig and other animals that may be hosts for the nematode. Invasive larvae for a given species may be obtained by the method of coproculture, for example by collecting feces from the animal infected with a given species of the nematode, and isolation of eggs from feces or their suspension for the purpose of breeding invasive larvae and infecting another non-human animal. Preferably, in order to obtain larvae in the invasive stage, feces are collected at a suitable interval from the time of contamination, mixed with water, and such slurry is applied to a moist paper placed on a Perti dish, incubated for a suitable time under appropriate temperature and light conditions selected for the given nematode species, and subsequently larvae are collected, preferably rinsing them off the medium several times with water.

The invasive form obtained in this way, preferably in the L3 stage, can be used to infect another non-human animal. After the time needed for the larvae to pass to the next stage, in which it colonizes the small intestine, preferably the L4 stage, the small intestine is isolated and placed in a medium under conditions allowing the larvae to escape into the medium. The larvae in the next stage obtained in this way, preferably in the L4 stage, can be suspended in a culturing medium and thus the solution of excretory-secretory proteins, in the form of a solution of the products secreted by these larvae in the culturing medium, can be obtained. Nematodes can be separated from the medium, for example, manually or by centrifugation.

The obtained larvae, preferably in the L4 stage, can also be shredded, for example, by sonication or homogenization, grinding. In this way, a solution containing somatic proteins of the nematode can be obtained.

The helminth in the adult form can be suspended in the culturing medium and thus the solution of excretory-secretory proteins, in the form of a solution of the products secreted by these parasites to the culturing medium, can be obtained. Nematodes can be separated from the medium, for example, manually or by centrifugation.

It is evident that subjecting the solution: the medium, wherein the adult forms and/or larvae in the L4 stage were cultured, containing a solution of excretory-secretory proteins, in a solution with the shredded larvae, for example by sonication or homogenization/grinding will result in obtaining a solution of somatic and excretory-secretory proteins of the helminth from this stage of development.

A person skilled in the art will understand that in order to *ex vivo* induce CD8+CD25+FOXP3-lymphocytes, preferably CD8+CD25+FOXP3-CD103- lymphocytes, solutions of somatic proteins and solutions of excretory-secretory proteins from different helminth species and/or from helminths in different stages of development: L1, L2, L3, L4, the pre-adult stage L5, preferably L4, of the nematode *Heligmosomoides polygyrus,* can be combined with each other in any way, or be used separately. Solutions of somatic and excretory-secretory proteins from the L4 stage of the helminth, particularly solutions of somatic proteins of the L4 stage of the nematode *H. polygyrus,* were found to be preferred.

The concentration of the solution of somatic and/or excretory-secretory proteins and/or protein fractions and/or recombinant proteins, the number of peripheral blood cells, culture time, and duration of stimulation may be different and can be easily determined experimentally by a skilled person. Typically, whole blood or peripheral blood mononuclear cells (PMCBs) isolated from a fresh sample, for example, heparinized blood by density gradient centrifugation, are cultured in the presence of different concentrations of the solution of somatic and/or excretory-secretory proteins of the parasite for 24-120 hours under conditions enabling their proliferation, preferably at 37°C, 5% $CO_2$ and 95% humidity, for example, according to the procedure described in Example 7.

After this time, leucocytes in the culture are routinely phenotyped and their viability is determined. A sterilely sorted population of live suppressor CD8+CD25+FOXP3- lymphocytes, more preferably CD8+CD25+FOXP3-CD103- (for specific identification of lymphocytes, additional sorting is performed using the marker CD3, i.e. the CD3+CD8+CD25+FOXP3-CD103- population is sorted out) is obtained by sterile sorting and in a therapeutically effective dose is administered to the patient, preferably autologously.

The *ex vivo* induced CD8+CD25+FOXP3-lymphocytes, more preferably CD8+CD25+FOXP3-CD103- lymphocytes with a specific immunosuppressive function, are transferred to the patient, preferably autologously, and they replace or support the minimized pharmacological therapy.

A therapeutically effective dose is to be understood as such an amount of the active ingredient, which are the CD8+CD25+FOXP3- lymphocytes, more preferably the CD8+CD25+FOXP3-CD103- lymphocytes obtained by the method for *ex* vivo induction which produces the desired therapeutic effect, for example inhibition, alleviation, slowing down or curing of the autoimmune disease.

The therapeutically effective dose administered systemically depends on the age, body weight, state of health, sex, diet, time of administration and severity of the disease state. The exact dose can be verified by a person skilled in the art. The induced CD8+CD25+FOXP3- cells, more preferably the CD8+CD25+FOXP3-CD103- lymphocytes obtained by the method for *ex vivo* induction of lymphocytes with the CD8+CD25+FOXP3- phenotype, more preferably the CD8+CD25+FOXP3-CD103- lymphocytes according to the invention, can be administered by one or more routes of administration using one or more of the various methods known in the art. The preferred routes of described administration include intravenous, intrathecal or other parenteral routes of administration by injection or infusion.

An individual can be an animal, preferably a mammal, more preferably a human. The solution of somatic and/or excretory-secretory proteins from a helminth parasite and/or functional protein fractions of said solution and/or recombinant proteins with amino acid sequence identical in at least 70% with the proteins from the abovementioned solution or retaining the activity of the protein of the abovementioned solution, for described use or also the administration of a solution of helminth proteins to an individual suffering from or an individual suspected to be suffering from an autoimmune disease related to the presence of autoreactive T lymphocytes, in order to increase the amount and activation of CD8$^+$CD25$^+$FOXP3$^-$ lymphocyte population, more preferably the CD8$^+$CD25$^+$FOXP3-CD103$^-$ lymphocytes with suppressive properties within this individual or administration to an individual suffering from or an individual suspected to be suffering from an autoimmune disease related to the presence of autoreactive T lymphocytes a solution of helminth proteins in order to increase the amount and activation of CD8$^+$CD25$^+$FOXP3$^-$ lymphocyte population, more preferably the CD8$^+$CD25$^+$FOXP3-CD103-lymphocytes with suppressive properties in this individual, can occur in a pharmaceutical composition that additionally contains a pharmaceutically acceptable carrier or diluent and optionally other active ingredients and/or excipients, e.g. using a drug delivery system that is safe for gastric mucosa, e.g., lime-alginate-chitosan hydrogel microspheres (Matricardi et al., 2008). It is also described a method of treatment or prevention of diseases related to the presence of autoreactive T lymphocytes, in particular an autoimmune disease in an individual, wherein the individual is administered lymphocytes with the CD8$^+$CD25$^+$FOXP3$^-$ phenotype, more preferably the CD8$^+$CD25$^+$FOXP3-CD103$^-$ lymphocytes induced by the method for *ex vivo* induction according to the invention.

In a preferred described method of treatment or prevention of diseases related to the presence of autoreactive T lymphocytes as described, the disease is selected from the following group: autoimmune diseases such as non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis.

In a preferred described method of treatment or prevention of diseases related to the presence of autoreactive T lymphocytes, the disease is ulcerative colitis and/or multiple sclerosis.

In a preferred described method of treatment or prevention of diseases related to the presence of autoreactive T lymphocytes, the individual is also administered at least one or more therapeutic agents used in the symptomatic treatment of the disease related to the presence of autoreactive T lymphocytes, wherein the administration is separated in time or concurrent.

In a preferred described method of treatment or prevention of diseases related to the presence of autoreactive T lymphocytes, the individual is also administered CD4$^+$CD25$^+$FOXP3$^+$ lymphocytes, previously induced *ex vivo.*

The description teaches a method for *in vivo* induction of lymphocytes with the CD8$^+$CD25$^+$FOXP3$^-$ phenotype, more preferably the CD8$^+$CD25$^+$FOXP3-CD103$^-$ lymphocytes in an individual diagnosed as suffering from or suspected to be suffering from an autoimmune disease related to the presence of autoreactive T lymphocytes, in particular an autoimmune disease, wherein the individual is also administered a solution of proteins of a helminth parasite and/or functional protein fractions of the abovementioned solution, and/or recombinant proteins with amino acid sequence identical in at least 70% with the proteins in the abovementioned solution, retaining the activity of proteins of the solution.

In preferred described method for *in vivo* induction of lymphocytes with the CD8$^+$CD25$^+$FOXP3-phenotype, more preferably the CD8$^+$CD25$^+$FOXP3-CD103$^-$ lymphocytes, the solution of somatic and/or excretory-secretory proteins of a helminth parasite and/or functional protein fractions of the abovementioned solution, comprise somatic and/or excretory-secretory proteins of a helminth parasite or proteins with amino acid sequence identical in at least 70% with the abovementioned proteins.

In preferred described method for *in vivo* induction of lymphocytes with the CD8$^+$CD25$^+$FOXP3-phenotype, more preferably the CD8$^+$CD25$^+$FOXP3-CD103$^-$ lymphocytes, the helminth parasites are larvae of the nematode *Heligmosomoides polygyrus,* including all the developmental stages - eggs, L1, L2, L3, L4 larvae, pre-adult stage L5, preferably L4 and adult forms of the nematode *Heligmosomoides polygyrus.*

In preferred described method for *in vivo* induction of lymphocytes with the CD8$^+$CD25$^+$FOXP3-phenotype, more preferably the CD8$^+$CD25$^+$FOXP3$^-$ lymphocytes, the disease is selected from the following group: autoimmune disease such as non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis.

In described method for *in vivo* induction of lymphocytes with the CD8$^+$CD25$^+$FOXP3$^-$ phenotype, more preferably the CD8$^+$CD25$^+$FOXP3$^-$ lymphocytes, the disease is ulcerative colitis and/or multiple sclerosis.

In described method for *in vivo* induction of lymphocytes with the CD8$^+$CD25$^+$FOXP3$^-$ phenotype, more preferably the CD8+CD25+FOXP3- lymphocytes, the individual is also administered at least one or more therapeutic agents used in the symptomatic and/or immunomodulating treatment of the disease related to the presence of autoreactive T lymphocytes such as, e.g., cholinolytic drugs, antidepressants, antibiotics, and immunomodulators such as beta-interferons, acting by increasing the number of regulatory CD4+CD25+FOXP3+ lymphocytes, wherein the administration is separated in time or concurrent.

In described method for *in vivo* induction of lymphocytes with the CD8+CD25+FOXP3- phenotype, more preferably the CD8+CD25+FOXP3- lymphocytes, the individual is also administered CD4+CD25+FOXP3+ lymphocytes, previously induced *ex vivo.*

The described use of lymphocytes with the CD8+CD25+FOXP3- phenotype, more preferably CD8+CD25+FOXP3-CD103- induced by the method for *ex vivo* induction according to the invention for the production of a medicine for use in treatment or prevention of diseases related to the presence of autoreactive T lymphocytes, in particular an autoimmune disease in an individual.

In desccibed use of lymphocytes with the CD8+CD25+FOXP3- phenotype, more preferably the CD8+CD25+FOXP3-CD103- lymphocytes for the production of a medicine for described use, the disease is selected from the following group: autoimmune diseases such as non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis.

In described aspect of using lymphocytes with the CD8+CD25+FOXP3- phenotype, more preferably the CD8+CD25+FOXP3-CD103- lymphocytes for the production of a medicine for described use, the disease is ulcerative colitis and/or multiple sclerosis.

There is described a use of a solution of somatic proteins and/or a solution of excretory-secretory proteins, from a helminth parasite and/or functional protein fractions of the abovementioned solution, and/or recombinant proteins with amino acid sequence identical in at least 70% with the proteins in the abovementioned solution, retaining the activity of proteins of the solution, for the production of a medicine for use in *in vivo* induction of lymphocytes with the CD8+CD25+FOXP3-phenotype, more preferably the CD8+CD25+FOXP3-CD103- lymphocytes in an individual, which is diagnosed as suffering from or suspected to be suffering from an autoimmune disease related to the presence of autoreactive T lymphocytes, in particular an autoimmune disease.

In described uses of the solution of somatic and/or excretory-secretory proteins and/or functional protein fractions and/or recombinant proteins, the solution of proteins of a helminth parasite and/or functional protein fractions of the abovementioned solution contain somatic and/or excretory-secretory proteins, from a helminth parasite or proteins with amino acid sequence identical in at least 70% with the proteins in the abovementioned proteins.

In described uses of the solution of somatic and/or excretory-secretory proteins and/or functional protein fractions and/or recombinant proteins, the helminth parasites are larvae of the nematode *Heligmosomoides polygyrus,* including all the developmental stages - eggs, L1, L2, L3, L4 larvae, pre-adult stage L5, preferably L4 and adult forms of the nematode *Heligmosomoides polygyrus.* In described uses of the solution of somatic and/or excretory-secretory proteins and/or functional protein fractions and/or recombinant proteins, the disease is selected from the following group: autoimmune disease such as non-specific bowel inflammation: ulcerative colitis, Crohn's disease; neurological diseases: multiple sclerosis, myasthenia, acute disseminated encephalomyelitis; rheumatoid arthritis, reactive arthritis, ankylosing spondylitis, psoriatic arthritis; skin diseases: psoriasis, alopecia areata; systemic connective tissue diseases: systemic lupus erythematosus, scleroderma, systemic vasculitis; thyroid diseases: Hashimoto's thyroiditis, Graves' disease; Addison-Biermer anemia, Sarcoidosis, coeliac disease, as well as Parkinson's disease, Alzheimer's disease, amyotrophic lateral sclerosis.

In described uses of the solution of somatic and/or excretory-secretory proteins and/or functional protein fractions and/or recombinant proteins, the disease is ulcerative colitis and/or multiple sclerosis.

***Advantage over the solutions known and proposed in the state of the art***

The disclosure is based on an alternative use of parasite-derived factors - helminth proteins, for the induction of CD8+CD25+FOXP3- lymphocytes; preferably the CD8+CD25+FOXP3-CD103-lymphocytes, *ex vivo.* This will allow to utilize the very high immunosuppressive potential of nematodes, without applying a biological therapy with live worms, as proposed in *helminthic therapy.* The solution proposed involves isolation of autologous lymphocytes present in the organism of an individual suffering from or an individual suspected to be suffering from an autoimmune disease related to the presence of autoreactive T lymphocytes, and subjecting them to *ex vivo* activation using a solution of helminth proteins, containing somatic or excretory-secretory proteins from this nematode, preferably with antigens of L4 larvae of *H. polygyrus,* in order to increase the amount and activation of CD8+CD25+FOXP3-lymphocyte population, more preferably the CD8⁺CD25⁺FOXP3⁻CD103⁻ lymphocytes with suppressive properties or administration to an individual suffering from or an individual suspected to be suffering from an autoimmune disease related to the presence of autoreactive T lymphocytes a solution of helminth proteins in order to increase the amount and activation of CD8⁺CD25⁺FOXP3⁻lymphocyte population, more preferably the CD8⁺CD25⁺FOXP3⁻CD103⁻ lymphocytes with suppressive properties in this individual.

The disclosure provides a proposal for a safe, non-toxic, antigen-specific and personalized therapy. Currently, the treatment of autoimmune and neurodegenerative diseases most commonly involves the use of a pharmacological therapy - non-steroidal anti-inflammatory drugs (NSAIDS) and glucocorticoids, which are symptomatic and do not address the underlying causes of the disease. They require, however, a close monitoring of the patient, not only in the aspect of peripheral blood morphology, but also in the liver and kidney function and they cause a number of drug-induced complications.

The proposed use of the induced CD8⁺CD25⁺FOXP3⁻ lymphocytes, more preferably the CD8⁺CD25⁺FOXP3⁻CD103⁻ lymphocytes according to the invention or obtained by the described method for *ex vivo* induction of the CD8⁺CD25⁺ lymphocytes, takes into account the cause of the autoimmune disease, related to the misrecognition of own body cells. This greatly outweighs the strategies for the treatment of autoimmune diseases aimed at neutralizing or blocking the activity of single proinflammatory cytokines such as, among others, TNF-α. Such an approach does not take into account the causes of the disease, which greatly limits the potential therapeutic effect. As it was described herein, it was unexpectedly demonstrated on a mouse model, that a therapeutic effect of the transfer of suppressor CD8⁺CD25⁺FOXP3⁻ lymphocytes, more preferably the CD8⁺CD25⁺FOXP3⁻CD103⁻ lymphocytes induced by the method according to the invention is much stronger than that described in the art for Treg CD4⁺CD25⁺FOXP3⁺ lymphocytes. To date, Treg CD4⁺CD25⁺FOXP3⁺ have been regarded as regulatory lymphocytes with the most potent immunosuppressive potential, which limit the excessive inflammatory response and which are the therapeutic agent in autoimmune and neurodegenerative diseases (Gonzalez-Rey et al., 2006). Lymphocytes with the CD8⁺CD25⁺FOXP3⁻ phenotype, preferably CD8⁺CD25⁺FOXP3⁻CD103⁻ or obtained by the method for *ex vivo* induction of lymphocytes with the CD8⁺CD25⁺FOXP3⁻phenotype, preferably the CD8⁺CD25⁺FOXP3⁻CD103⁻ lymphocytes according to the invention provide better effects in the prevention or treatment of autoimmune diseases than the currently known methods.

As it was demonstrated, suppressor CD8⁺CD25⁺FOXP3⁻ lymphocytes, preferably CD8⁺CD25⁺FOXP3⁻CD103⁻ lymphocytes, activated by a solution of proteins of a parasite have a strong therapeutic effect in a many times lower dose, than that proposed in the art for clinical use of Treg CD4⁺CD25⁺FOXP3⁺. This indicates a high suppressive potential of the activated CD8⁺CD25⁺ lymphocytes compared to that of Treg CD4⁺CD25⁺FOXP3⁺ described in the state of the art, and provides an increased possibility of obtaining *ex vivo* a sufficient number of lymphocytes to achieve an *in vivo* therapeutic effect.

The lymphocytes described herein and obtained in a culture by a method for *ex vivo* induction of CD8⁺CD25⁺ lymphocytes, do not express the FOXP3 transcription factor (CD8⁺CD25⁺FOXP3⁻), more preferably are CD8⁺CD25⁺FOXP3⁻CD103⁻, and are characterized by high viability, which indicates their *in vivo* applicability without loss of the suppressive potential.

The key problem with the use of CD4⁺CD25⁺FOXP3⁺ Treg cells in therapy is optimization of the method for culturing thereof under *ex vivo* conditions, because the cytokines IL-2 and TGF-β used for stimulation, differentiate the naïve CD4⁺CD25⁻ cells into cells with the CD4⁺CD25⁺FOXP3⁺ phenotype, but they do not provide stability during adaptive transfer.

The method for inducing CD8⁺CD25⁺FOXP3⁻ lymphocytes, preferably CD8⁺CD25⁺FOXP3⁻CD103⁻ according to the invention is much safer than *in vivo* induction of regulatory CD4⁺CD25⁺FOXP3⁺ lymphocytes, e.g. in the course of multiple sclerosis after steroid therapy or in the proposed method of administration of non-antigen-specific vaccines (by a DNA plasmid encoding an autoantigen in multiple sclerosis), inducing generation of regulatory cells in the body. Autologous CD8⁺CD25⁺FOXP3⁻ lymphocytes, preferably CD8⁺CD25⁺FOXP3⁻CD103⁻, are present in the body during parasitic infections and they have natural suppressive properties, therefore their therapeutic application is not associated with a significant risk. The use of *ex vivo* induced CD8⁺CD25⁺FOXP3⁻, more preferably CD8⁺CD25⁺FOXP3⁻CD103⁻, may constitute a non-toxic, biological targeted therapy restoring normal function of excessive immune response. It is recommended that administration is performed intravenously, intrathecally or by other parenteral routes by injection or infusion of lymphocytes, what guaranties 100% bioavailability.

Therapeutic use of the induced CD8⁺CD25⁺FOXP3⁻ lymphocytes, preferably CD8⁺CD25⁺FOXP3⁻CD103⁻ as obtained by the method for induction according to the disclosure eliminates or limits the dosing of medication in patients and the occurrence of drug-induced complications and harmful interactions resulting from the commonly used polypharmacotherapy.

In the proposed application, the invention eliminates the possibility of hypersensitivity, including contact hypersensitivity in the proposed methods for induction of tolerance through the skin, using, e.g. myelin protein in multiple sclerosis (Askenase et al., 2004).

Finally, the proposed therapeutic solution does not require the costs associated with pharmacodynamic and pharmacokinetic studies and the development and preparation of carriers, which will protect the compound against rapid release, such as controlled release formulations and microencapsulation delivery systems that may be required for the therapy using nematode proteins administered orally. Such formulations, necessary in the case of oral administration of medicines, are generally known to specialists. In addition, the disclosed therapy is directed to a broad spectrum of patients. There is no risk of a different response of the patient to the therapy due to *in vivo* changes in pharmacodynamics and pharmacokinetics.

Publications cited in the description, and the references given therein, are in their entirety incorporated herein as references. For a better understanding of the invention, it has been illustrated in the embodiments and in the accompanying figures.

***Brief description of figures***

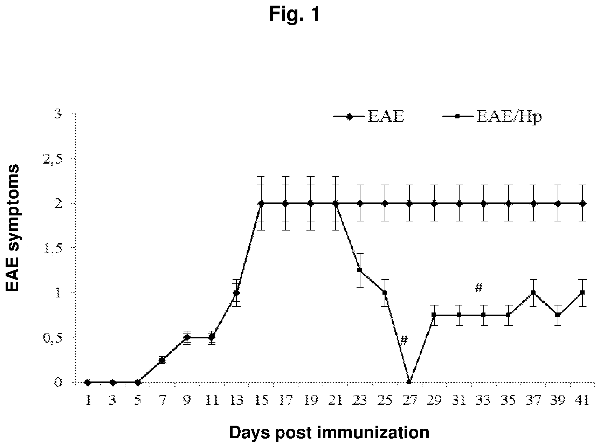

**FIG 1** shows the effect of infection with the *H. polygyrus* nematode on the EAE symptoms in female C57Bl6 mice. #- statistically significant differences ($\pm$ SE) compared to the mice with EAE on the same day after infection. EAE-animals with EAE, not infected with the parasite, EAE/HP-animals with EAE, infected with the parasite.

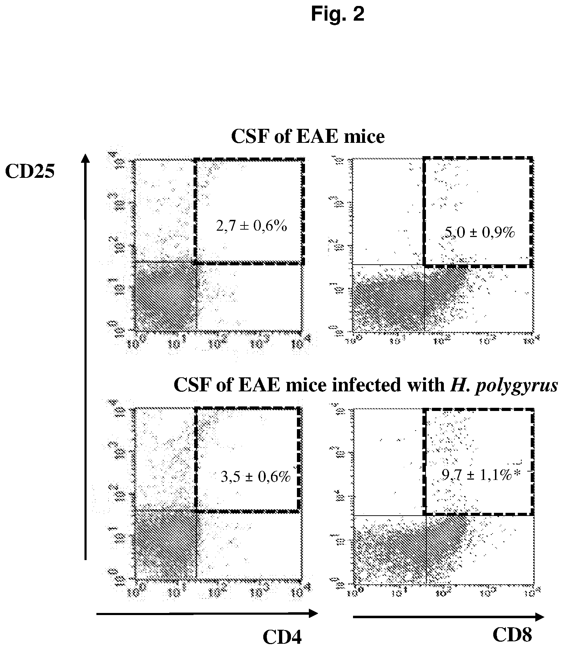

**FIG 2** shows the effect of 6 days of infection with the *H. polygyrus* nematode in mice with EAE on the percentage of $CD4^+CD25^+FOXP3^+$ and $CD8^+CD25^+FOXP3^-$ lymphocyte population in cerebrospinal fluid (CSF). * A statistically significant difference ($\pm$ SE) compared to the same population of lymphocytes in not-infected mice with EAE.

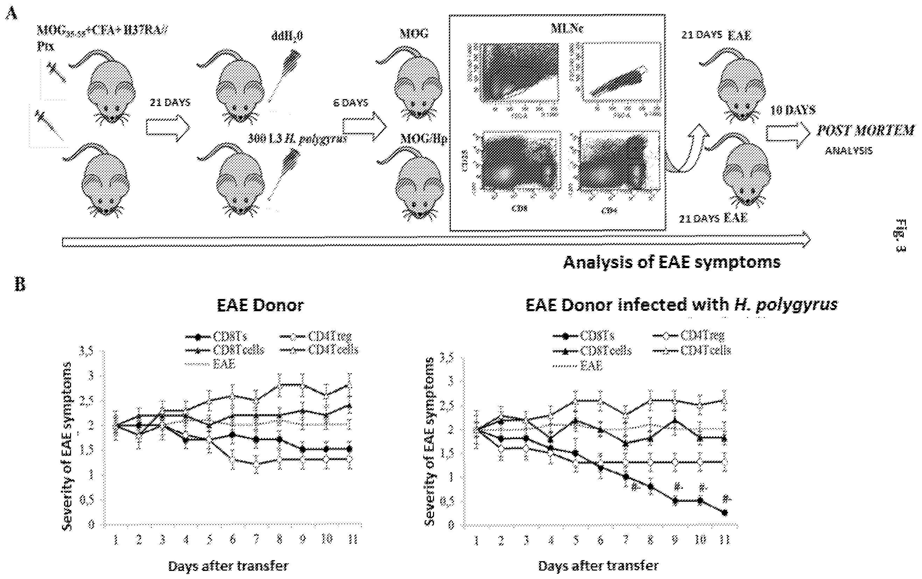

**FIG 3** shows the effect of the transfer of $CD4^+$ and $CD8^+$ subpopulations of lymphocytes, isolated from mesenteric lymph nodes from donors with EAE and donors with EAE infected with the *H. polygyrus* nematode on the 6th day of infection on the course of EAE. A- Outline of the experiment: female mice of the C57BU6J strain at the age of 8 weeks were immunized according to the method described in Example 1. On the 21st day after immunization, the following lymphocytes, cytometrically sorted, were administered to the animals: $CD4^+CD25^-$ (CD4Tcells) or $CD4^+CD25^+FOXP3^+$ (CD4Treg) or $CD8^+CD25^-$ (CD8Tcells) or $CD8^+CD25^+FOXP3^-$ (COSTs) from donors not infected (donor with EAE) or infected with the nematode (donor with EAE infected with *H. polygyrus),* respectively. B- Mice were weighed and observed for increased EAE symptoms (clinical score). #- statistically significant differences ($\pm$ SE).

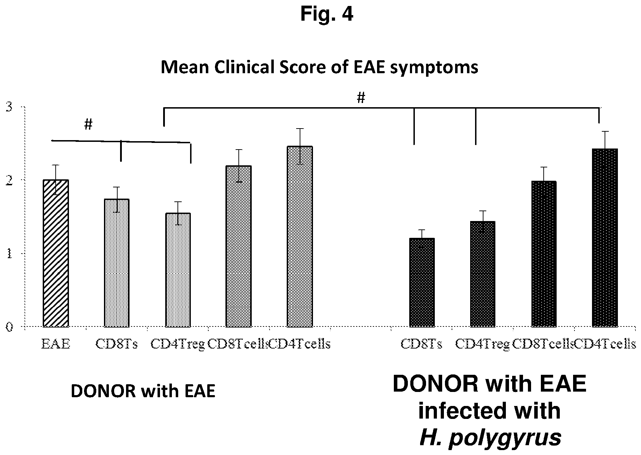

**FIG 4** shows the mean increase in the EAE symptoms (the mean clinical score) in mice after the transfer of an adequate subpopulation of lymphocytes: $CD4^+CD25^-$ (CD4Tcells) or $CD4^+CD25^+FOXP3^+$ (CD4Treg) or $CD8^+CD25^-$ (CD8Tcells) or $CD8^+CD25^+FOXP3^-$ (CD8Ts) from donors not infected (donor with EAE) or infected with the nematode (donor with EAE infected with *H. polygyrus),* respectively. #- statistically significant differences ($\pm$ SE).

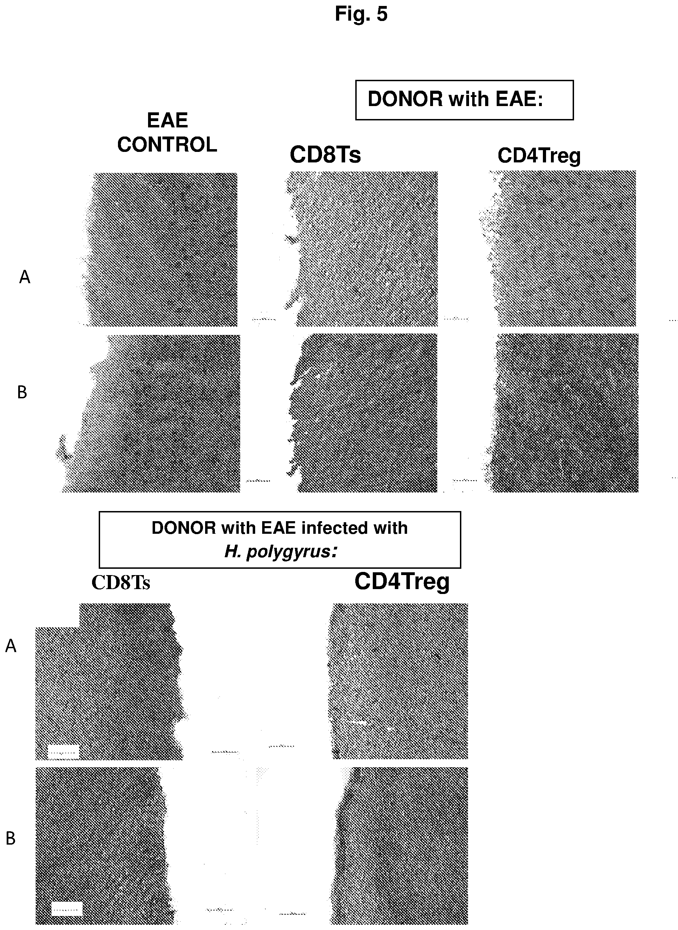

**FIG 5** shows the changes in the brain of mice on the 11th day after the transfer of lymphocytes: $CD4^+CD25^+FOXP3^+$ (CD4Treg) and $CD8^+CD25^+FOXP3^-$ (CD8Ts) from donors not infected (donor with EAE) or infected with the nematode (donor with EAE infected with *H. polygyrus),* respectively. A- Brain sections were stained with hematoxylin and eosin (H&E), in order to visualize the infiltration of leucocytes to the brain, and B- eriochrome cyanine R (solochrome cyanine) dyeing the myelin fibers blue in order to evaluate the demyelination process. Visible white demyelination plaques- damage to the myelin sheath of the axons. *Scale*- 100 $\mu$m.

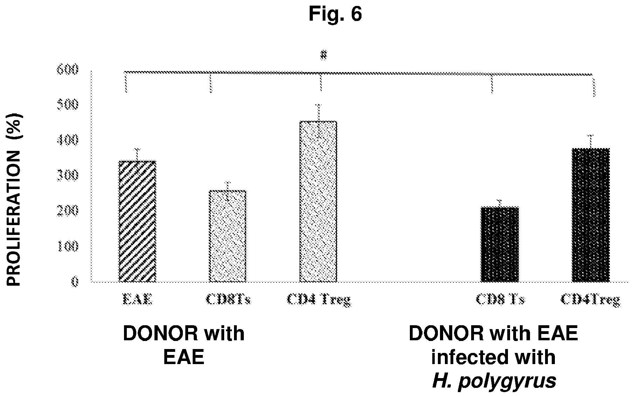

**FIG 6** shows the effect of the transfer of $CD4^+CD25^+FOXP3^+$ (CD4Treg) and $CD8^+CD25^+FOXP3^-$(COSTs) lymphocytes obtained from donors not infected (donor with EAE) or infected with the nematode (donor with EAE infected with *H. polygyrus),* respectively, on the proliferation ability of $MOG_{35-55}$ - specific T lymphocytes from the cerebrospinal fluid of recipients in the *in vitro* culture. #- statistically significant differences ($\pm$ SE).

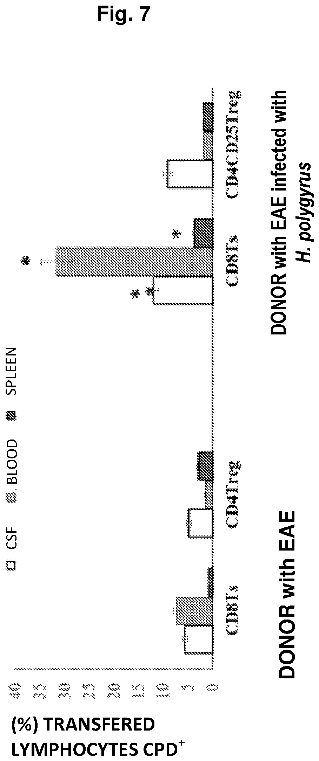

**FIG 7** shows the location and percentage of $CD4^+CD25^+FOXP3^+$ (CD4Treg) and $CD8^+CD25^+FOXP3^-$ (COSTs) lymphocytes transferred from donors among the lymphocytes with the same phenotype in the tissues of the recipients. The transferred cells were labeled with a fluorescent dye Dye eFluor 670 - CPD, which allows for *in vivo* identification of cells. *Statistically significant results - comparison of the same tissues between recipient groups to which lymphocytes with the same phenotype were transferred.

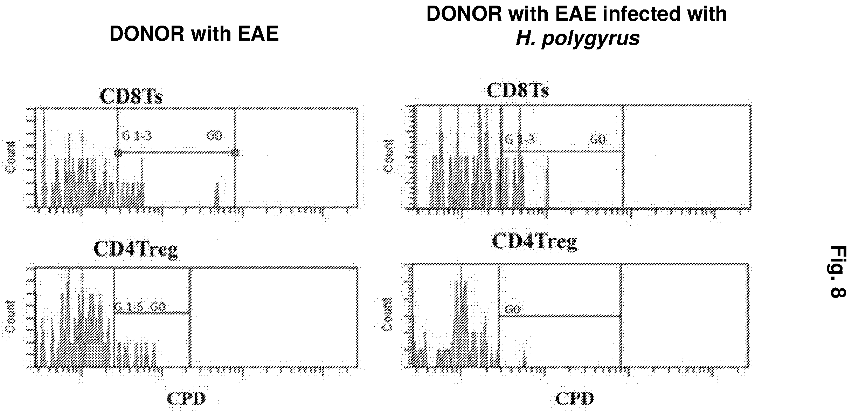

**FIG 8** shows the number of cell divisions (successive generations) of $CD4^+CD25^+FOXP3^+$ (CD4Treg) and $CD8^+CD25^+FOXP3^-$ (CD8Ts) lymphocytes transferred from donors in the cerebrospinal fluid of the recipient. Populations of cells labeled with the CPD fluorescent dye after successive divisions (successive generations; G1-) were determined based on the twofold decrease in the intensity of CPD fluorescence compared to the fluorescence of the previous population. The range of population of mother cells (G0) was determined based on the fluorescence intensity.

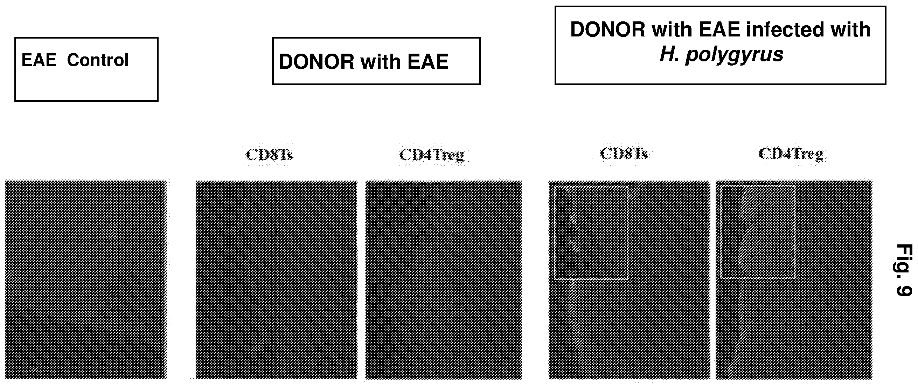

**FIG 9** shows infiltration of CD4$^+$CD25$^+$FOXP3$^+$ (CD4Treg) and CD8$^+$CD25$^+$FOXP3$^-$ (CD8Ts) lymphocytes, labeled with the CPD fluorescent dye, transferred from donors not infected (donor with EAE) or infected with the nematode (donor with EAE infected with *H. polygyrus*), respectively, in the brain of recipients. The analyzes were performed using the Leica system- DM5000 B 337 fluorescence microscope, DFC350FX camera and LAS AF software. Magnification x20.

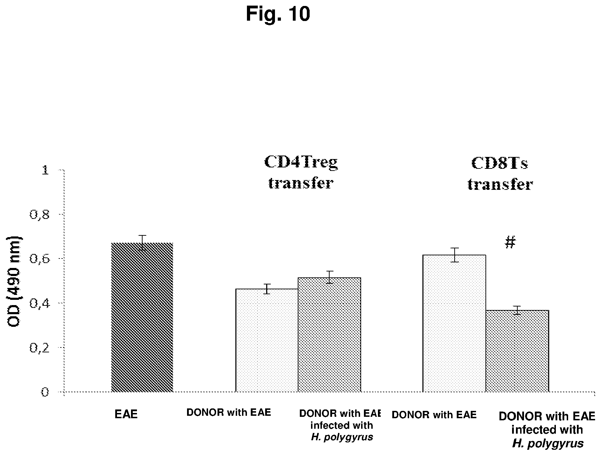

**FIG 10** shows the effect of CD4$^+$CD25$^+$FOXP3$^+$ (CD4Treg) and CD8$^+$CD25$^+$FOXP3$^-$ (CD8Ts) lymphocytes isolated from mice not infected (donor with EAE) or infected with the nematode (donor with EAE infected with *H. polygyrus*), respectively, on the proliferation ability of MOG-specific T lymphocytes from the cerebrospinal fluid of mice with EAE in the *in vitro* mixed lymphocyte culture (MLR). CSF leucocytes isolated from mice with EAE were stimulated with 5mM MOG$_{35-55}$ of cultured without stimulation in the presence of the abovementioned lymphocytes for 72 h. The results are shown as the value of optical density (OD). #- Statistically significant results compared to the control group (EAE, ± SE).

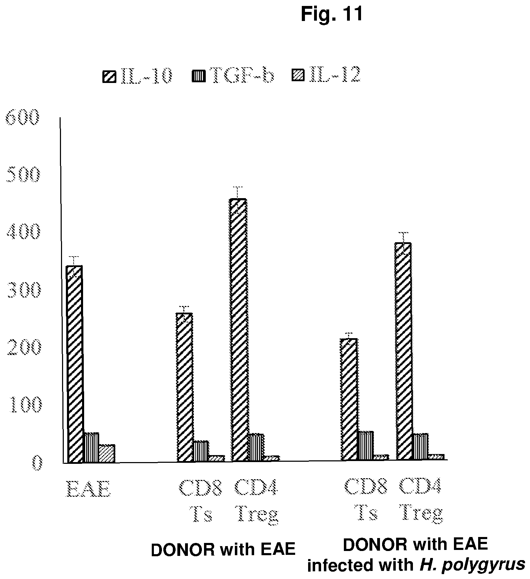

**FIG 11** shows the effect of CD4$^+$CD25$^+$FOXP3$^+$ (CD4Treg) and CD8$^+$CD25$^+$FOXP3$^-$ (CD8Ts) lymphocytes isolated from mice not infected (donor with EAE) or infected with the nematode (donor with EAE infected with *H. polygyrus*), respectively, on the production of IL-10 and TGF-β by cerebrospinal fluid leucocytes of mice with EAE in the *in vitro* mixed lymphocyte culture (MLR).

CSF leucocytes isolated from mice with EAE were stimulated with 5mM MOG$_{35-55}$ of cultured without stimulation in the presence of the abovementioned lymphocytes for 72 h. The cytokine level was determined by the ELISA test. #- Statistically significant results compared to the control group (EAE, ± SE).

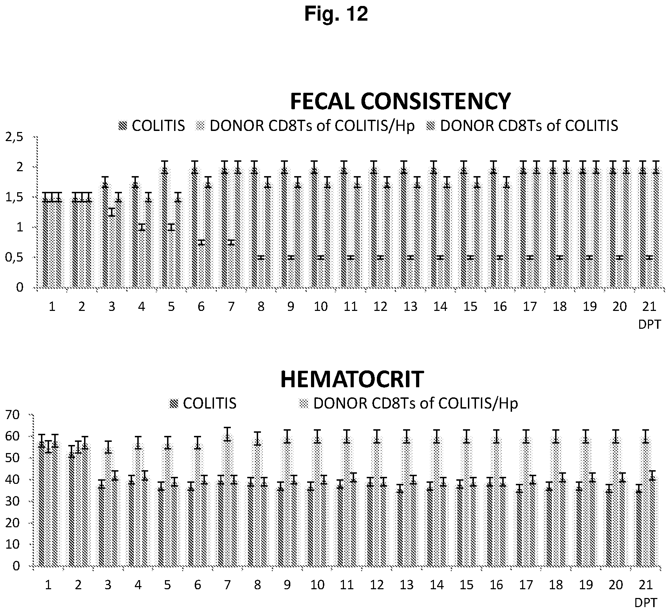

**FIG 12** shows the course of colitis in mice transferred with CD8$^+$CD25$^+$ (COSTs) lymphocytes, isolated from mesenteric lymph nodes of donors with colitis (Colitis) and donors with colitis infected with the *H. polygyrus* nematode (COLITIS/Hp) on the 6th day of infection of mice with induced colitis. Every day mice were examined for aggravation of the symptoms of colitis based on fecal consistency and hematocrit level. The Colitis group vs. the CD8$^+$CD25$^+$ (COSTs) recipient with COLITIS/Hp - statistically significant results (± SE).

*Examples*

In the following examples, unless it was otherwise indicated, manufacturers' instructions for specific materials and methods were followed. Concentrations of all the stimulants and cells in the cultures, as well as culturing time (culture duration) was determined based on preliminary experiments.

**Example 1.**

**Infection with a *H. polygyrus* nematode induces lymphocytes of a CD8$^+$CD25$^+$ population in cerebrospinal fluid of mice with autoimmune encephalomyelitis, EAE**

Mice of the C57Bl6 strain, females at the age of 8 weeks, were actively immunized in order to induce EAE by administering MOG$_{35-55}$ glycoprotein (of purity > 95%) in the amount of 200 μg per mouse suspended in 50 μL of complete Freund's adjuvant (CFA) enriched with 300 μg of *Mycobacterium tuberculosis* strain H37RA lysate. 100 μL of the homogenate (mixture) was administered subcutaneously (s.c.). On the day of immunization and 48 h later, 400 ng of *Bordetella pertussis* toxin (PTx) was administered intraperitoneally in 100 μl of BPS buffer with the pH of 7.2. Non-immunized, control mice were administered PBS 7.2 buffer alone, in the same way.

The animals were observed every day and aggravation of EAE neurological symptoms was evaluated. A clinical evaluation was performed with a three-step scale demonstrating a degree of mobility impairment correlating with the progression of the disease: 0- no symptoms; 1- slight tail flaccidity and/or paresis of front limbs and/or hind limbs; 2- distinct tail flaccidity and/or paresis of front limbs and/or hind limbs; 3- intense tail paralysis and/or paralysis of front limbs and/or hind limbs. After 19 days after immunization, an aggravation of symptoms was observed. On the 21st day after immunization animals were infected with a dose of 300 L3 stage *H. polygyrus* larvae in 200 μl of drinking water by an esophageal probe. Animals of the group of EAE mice not infected with the nematodes were administered 200 μl of drinking water. Aggravation of clinical symptoms in animals was evaluated.

Cerebrospinal fluid (CSF, approx. 20 μL) was collected on the 6th day after infecting the animals by using a capillary inserted between the occipital nodules and top vertebra of the spine. CSF cells were phenotypically analyzed based on surface markers with the use of rat, anti-mouse antibodies conjugated with fluorescent dyes: rat allophycocyanin (APC)-anti-CD25, fluorescein isothiocyanate (FITC)-anti-CD8, peridinin-chlorophyll-protein complex (PercP)-anti-CD4 and the level of intracellular transcription factor FOXP3 was identified with the use of antibody: pphycoerythrin (PE)-anti FOXP3 in a FACSCalibur flow cytometer (BD). Analysis was performed using LYSIS II Software (BD).

The experiment was repeated 3 times on groups of 5 animals.

As illustrated in **FIG 1,** remission of EAE clinical symptoms in mice infected with the *H. polygyrus* nematode occurs from the second day of infection. On the 6th day of the infection, when nematode larvae in the L4 stage are present in the muscles of small intestine, a complete lack of EAE symptoms is observed. On the 6th day after nematode infection the lack of EAE clinical symptoms correlated with an increase in the percentage of lymphocytes with $CD8^+CD25^+$ phenotype from the mean level of $5.0 \pm 0.9\%$ for the EAE mouse to $9.7 \pm 1.1\%$ for the EAE mouse infected with *H. polygyrus,* which is shown in **FIG 2.** Mice infected with nematodes did not increase the percentage of $CD8^+CD25^+$ cells, positive in the terms of the FOXP3 transcription factor. These cells also did not show increased FOXP3 expression.

### Example 2.

### Adaptive transfer of $CD8^+CD25^+FOXP3^-$ lymphocytes from diseased mice, infected with *H. polygyrus* inhibits symptoms and inflammation in EAE

The recipients were mice, females at the age of 8 weeks of the C57Bl6 strain, the donors were Foxp3$^{gfp}$ knock-in mice transgenic for FoxP3-GFP of the C57Bl6 strain, because the transcriptional FoxP3 factor cannot be labeled in live mice. As in Example 1 mice of the C57Bl6 strain, females at the age of 8 weeks, were actively immunized in order to induce EAE. Non-immunized, control mice were administered PBS 7.2 in the same way. The animals were observed every day and aggravation of EAE neurological symptoms was evaluated, as in Example 1. On the 21st day after immunization animals were infected with a dose of 300 L3 stage *H. polygyrus* larvae, as in Example 1. Animals from the group of EAE mice not infected with the nematodes were administered 200 μl of drinking water. Aggravation of clinical symptoms in animals was evaluated. Mean intensity of symptoms was determined (mean clinical score, **FIG** 4).

From mice-donors: with EAE (on the 27th day of immunization) and mice-recipients with EAE infected with *H. polygyrus* (on the 27th day of immunization and on the 6th day after the nematode infection) lymph mesenteric nodes were isolated, a cell suspension was obtained and cells were labeled with antibodies against the CD4, CD25 and CD8 molecules on cells: peridinin-chlorophyll-protein complex (PercP)- anti-CD4, pphycoerythrin (PE)-anti-CD8 and PE-Cy-7-anti-CD25 and the level of intracellular transcription factor FOXP3-GFP was identified. Labeled cells were analyzed and lymphocytes were sorted by subpopulations $CD4^+CD25^+FOXP3^+$ (CD4Treg) and $CD8^+CD25^+FOXP3^-$ (COSTs), $CD4^+CD25^-$ (CD4Tcells) and $CD8^+CD25^-$ (CD8Tcells) using FACSAria Sorter cell sorter (BD Biosciences). Purity of the sort was on average 97%, cell viability 98%. Sorted subpopulations were labeled with a fluorescent dye Dye eFluor 670 (CPD, eBioscience), which is non-toxic and allows the tracking of cell populations. Stained cells suspended in 100 μL PBS with pH of 7.2 in the amount corresponding to the cell size of the given subpopulation in mice lymph mesenteric nodes (that is as shown in **Table 1** below):

**Table 1.** Cell numbers of the particular lymphocyte subpopulations in lymph mesenteric nodes of mice.

| Donor | $CD8^+CD25^+FOXP3^-$ | $CD4^+CD25^+FOXP3^+$ | $CD8^+CD25^-$ | $CD4^+CD25^-$ |
|---|---|---|---|---|
| **From EAE** | $1.5 \times 10^4$ | $6 \times 10^4$ | $42 \times 10^4$ | $60 \times 10^4$ |
| **From EAE on the 6 day after infection with *H. polygyrus*** | $0.3 \times 10^4$ | $9 \times 10^4$ | $48 \times 10^4$ | $75 \times 10^4$ |

Lymphocytes $CD8^+CD25^+FOXP3^-$, $CD4^+CD25^+FOXP3^+$, $CD8^+CD25^-$, $CD4^+CD25^-$ of the donor were transferred - carried in injection into the tail vein of EAE mice on the 21st day of immunization. Control animals were administered by tail vein an injection of 100 μL of PBS with pH of 7.2. Animals were weighed and aggravation of clinical symptoms was evaluated every day.

Animals were evaluated *post mortem* 11 days after the transfer. Animal brains were isolated from the skull and kept for 24 hours in 10% of sucrose solution. After this time tissue was transferred into 20% for 24h and then into 30% of sucrose solution for another 24h. Frozen sections of thickness of 8 μm were made after 72h. Sections were observed and analyzed for the presence of labeled donor's CPD-lymphocytes using Leica DM5000 microscope and then standard dyeing with Cole's hematoxylin and acidic eosin Y was made in order to the evaluate level of leukocytes or standard dyeing with solochrome cyanine R dyeing myelin fibers blue was performed in order to evaluate the demyelination process. The preparations were analyzed in light microscope and archived.

Cerebrospinal fluid was collected from the recipients, as in the 1st example. Blood was drawn from the heart. Spleen was isolated and cell suspension was obtained. The number and viability of cells in the organs were analyzed. Cells were cytometrically analyzed in order to determine the percentage and proliferation of cells of transferred donors-CPD (Dye eFluor 670)- positive. Populations of cells after successive divisions (successive generations, G) were determined based on t twofold decrease in CPD fluorescence intensity relative to the fluorescence level of the previous population.

To evaluate activity and specificity of recipients lymphocytes after transfer, PMR lymphocyte proliferation capacity was examined. $2.5 \times 10^5$ PMR cells in the presence of 5 $\mu$M MOG$_{35-55}$ protein or antibodies anti-CD3, anti-CD28 (0.5 $\mu$g/ml) were incubated (in 6 repetitions) in 96-well plates in total volume of 100 $\mu$l of RPMI 1640 culture medium with 5% of inactivated bovine serum (FBS), penicillin (100 U / ml), streptomycin (100 $\mu$g / ml) and L-glutamine (2 mM) at the temperature of 37°C in 5% of CO$_2$ for 72h. Proliferation was assessed by a colorimetric method using the CellTiter 96 AQueous One Solution Cell Proliferation Assay (Promega).

Relative proliferation of lymphocytes was determined according to the equation:

$$Proliferation\ (\%)=(OD_{MOG}/OD_M)\ x\ 100.$$

where OD$_{MOG}$ is the optical density (OD) of cells stimulated with MOG$_{35-55}$ and OD$_M$ indicates the OD of non-stimulated cells cultured in cell medium.

As shown in **FIG 3** transfer of CD8$^+$CD25$^+$FOXP3$^-$lymphocytes from EAE animals on the 6th day of the *H. polygyrus* nematode infection leads to severe inhibition of EAE symptoms. Observed effects of EAE symptoms reduction in animals transferred with CD8$^+$CD25$^+$FOXP3$^-$lymphocytes (in the amount of 3000) from EAE infected animals was stronger than in animals transferred with CD4$^+$CD25$^+$FOXP3$^+$ lymphocytes from EAE mice infected with the nematode (at the same or even 5-times higher dose) and much stronger than the effect observed in EAE animals administered with CD8$^+$CD25$^+$FOXP3$^-$ lymphocytes from mice not infected with the nematode. As shown in **FIG 4,** the difference in average cumulative clinical symptoms between the study groups is statistically significant.

As shown in **FIG 5** the transfer of CD8$^+$CD25$^+$FOXP3$^-$lymphocytes from EAE donors infected with the nematode significantly induced a reduction in the number of brain infiltrating lymphocytes and a decrease of the number and size of demyelinating lesions. This was confirmed by the results for proliferation of lymphocytes in the cerebrospinal fluid. As shown in **FIG 6** PMR lymphocytes of CD8$^+$CD25$^+$FOXP3$^-$ recipients from EAE donors infected with the nematode (EAE donor infected with *H. polygyrus*) proliferated less in response to MOG$_{35-55}$ antigen stimulation. However, no effect on non-specific T lymphocyte proliferation has been demonstrated with anti-CD3 and anti-CD28 antibodies TCR stimulation

As shown in **FIG 7** and **FIG 9** both CD8$^+$CD25$^+$FOXP3$^-$ and CD4$^+$CD25$^+$FOXP3$^+$ lymphocytes from EAE donors infected with the nematode (EAE donor infected with *H. polygyrus*) were infiltrating PM R. Whereas a much higher percentage of CD8$^+$CD25$^+$FOXP3$^-$ lymphocytes was found in other tissues - blood and spleen of EAE-induced mice **(FIG 7).** As shown in **FIG 8** the transferred CD8$^+$CD25$^+$FOXP3$^-$ lymphocytes of EAE donors infected with the nematode have shown an increased *in vivo* proliferation as measured by the number of subsequent generations of cells compared to other lymphocyte populations from donors infected and not infected with nematodes.

**Example 3.**

**Lymphocytes of the CD8$^+$CD25$^+$FOXP3$^-$ population inhibit proliferation of MOG-specific T lymphocytes.**

In order to evaluate activity of CD8$^+$CD25$^+$FOXP3$^-$lymphocytes, mixed lymphocyte culture (MLR) was performed. As corresponding cells, CD3$^+$ lymphocytes isolated from the cerebrospinal fluid of an EAE mouse on the 21st day after immunization, were used. CD3$^+$ lymphocytes were subjected to negative isolation using magnetic beads Dynal Mouse T cells Negative Isolation Kit *(Dynal* Biotech, *Invitrogen)*. CD8$^+$CD25$^+$FOXP3$^-$ and CD4$^+$CD25$^+$FOXP3$^+$ lymphocytes at the amount of $1.25 \times 10^4$ were added to $5 \times 10^4$ corresponding CD3$^+$ lymphocytes. Cells were cultured in 100 $\mu$L of the RPMI 1640 culture medium with 5% inactivated bovine serum (FBS), penicillin (100 U / ml), streptomycin (100 $\mu$g / ml) and L-glutamine (2 mM) for 72h in the presence of 5 $\mu$M of MOG$_{35-55}$ and/or antibodies anti-CD3, anti-CD28 (0.5 $\mu$g/ml). Lymphocyte proliferation was evaluated by a colorimetric method, as in example 2. In an enzyme immunoassay the level of regulatory cytokines: IL-10 and TGF-$\beta$ and IL-12p70 was analyzed in culture supernatants

Comparison of the ability of CD8$^+$CD25$^+$FOXP3$^-$ and CD4$^+$CD25$^+$FOXP3$^+$ lymphocytes from EAE donors infected and not infected with the nematode to induce MLR, indicated, as shown in **FIG 10,** that CD8$^+$CD25$^+$FOXP3$^-$ population inhibited lymphocyte proliferation in ex *vivo* culture the strongest. In the same MLR cultures under the influence of CD8$^+$CD25$^+$FOXP3$^-$ lymphocytes, no level increase of IL-10, TGF-$\beta$ and IL-12p70 was noted. As shown in **FIG 11,**

CD4+CD25+FOXP3+ population of EAE donors infected and not infected with the nematode inhibited lymphocyte proliferation in the presence of increased levels of IL-10.

**Example 4**

**Adaptive transfer of CD8+CD25+ lymphocytes from *colitis* mice, infected with *H. polygyrus* inhibits colitis symptoms**

Studies were conducted on male mice of the BALB/c strain at the age of 8 weeks. Inflammatory bowel disease in mice was induced with 0,5% solution of sodium sulfate dextran (DSS, 35-50 kDa, ICN Biomedicals Inc., OH, USA). DSS was administered in potable water from the 5th day before infecting the mice with 300 *H. polygyrus* larvae (L3) until the end of the experiment. Animals that were in the *colitis* mice group not infected with nematodes, were administered 200 $\mu$l of drinking water in the same way.

From donor mice: from *colitis,* which were administered DSS for 11 days and from donor mice from *colitis,* infected with *H. polygyrus* (on the 6th day of the infection, which were administered DSS for 11 days), lymph mesenteric nodes were isolated, a cell suspension was obtained and the cells were labeled with antibodies against the CD25 and CD8 molecules on the cells: anti-CD8 with phycoerythrin (PE) and PE-Cy-7-anti-CD25. Labeled cells were analyzed and lymphocytes of CD8+CD25+ subpopulations were sorted using FACSAria Sorter cell sorter (BD Biosciences). Purity of the sort was at average of 96%, cell viability 97%. Sorted cells in quantity of 3000 suspended in 100 $\mu$L PBS with pH of 7.2 were transferred by intravenous injection to the main tail vein of recipients with colitis. Control animals were administered 100 $\mu$L PBS with pH of 7.2 in an injection to the tail vein.

The animals were observed every day and aggravation of colitis symptoms was evaluated. Evaluation was done with three-step scale demonstrating a degree of fecal consistency disorder correlated with the severity of the disease: based of three-step scale: 0- no diarrhea-tight feces; 1-loose feces; 2- diarrhea -liquid feces. Hematocrit was evaluated with macromethod on a daily basis.

As shown in **FIG 12** transfer of CD8+CD25+ lymphocytes from *colitis* animals on the 6th day of the infection with *H. polygyrus* nematode leads to the strong inhibition of colitis symptoms and hematocrit increase. In recipients who have CD8+CD25+ lymphocytes transferred from non-infected animals, no signs of disease inhibition were observed

**Example 5**

***In vitro* induction of CD8+CD25+FOXP3 lymphocytes in peripheral blood of mice with EAE**

L3 stadium larvae of *H. polygyrus* nematode invasive for mice are obtained from coproculture, mice feces are collected between 2-12 week after infection and mixed with water. The resulting slurry is applied to damp blotting paper in a Petri dish and incubated at 21°C. After 7 days of culture the edge of the blotting paper is rinsed and collected larvae are washed several times with water and stored at 4°C until the mice infection.

Subsequently, mice of the BALB/c strain at the age of 8 weeks were infected with the dose of 300 *H. polygyrus* larvae of the L3 stadium suspended in 200 $\mu$l of water using an esophageal probe. On the 6th day after the infection mice small intestines were isolated and placed in culture medium (RPMI 1640 + GlutaMAX, Gibco). The intestines were incubated at 37°C for a minimum of 1 to 2 h; at this time L4 larvae leave the intestinal wall and get out to the medium. Larvae were collected, counted and frozen in PBS pH 7.2, at -80°C.

A solution of somatic nematode proteins was obtained by fragmentation of nematodes by sonication. The nematode suspension was thawed and shredded with ultrasounds, at 4°C for 6 min. in the 20 s ON: 20 s OFF mode. Samples were centrifuged in 1000 g for 15 min at 4°C and the level of protein in the supernatant was determined spectrophotometrically. The protein solution was sterilized by passing through a filter with a pore size of 0.22 $\mu$m, portioned and stored at -80°C

On the 21st day after immunization, blood from the EAE mice was drawn from the heart by vacuum method to the Vaccuette tubes with heparin sodium in an amount of 14 I.U./ml of blood. Samples were stored at room temperature. Peripheral blood mononuclear cells (PBMC) was obtained by density gradient centrifugation. PBMCs were suspended in RPMI 1640 medium enriched with 5% of the fetal calf serum and penicillin/gentamicin (40mg/ml). The number of cells was determined using the Bürker chamber and the viability was determined using the trypan blue absorption assay. Such prepared PBMCs with cell viability> 90% were used for *in vitro* studies. Cells were placed in wells of flat-bottomed culture microplates at a density of 100,000 cells per well and cultured *ex vivo* in the presence of a solution of somatic L4 *H. polygyrus* larvae proteins in different concentrations 5 $\mu$g; 2.5 $\mu$g; 1.25 $\mu$g; 0.625 $\mu$g; 0.312 $\mu$g; 0.156 $\mu$g; 0.078 $\mu$g; 0.039 $\mu$g in total volume of the medium of 200 ml. Each culture was run in at least triplicates. Cultures were run for 120 hours, at the temperature of 37°C, in an atmosphere of 90% humidity, containing 95% air and 5% $CO_2$. After the incubation was completed, the cells were harvested from the wells and their viability was determined with Muse Annexin V & Dead Cell Kit in the cell analyzer MUSE (Milipore) and were phenotyped based on surface markers using antibodies: anti-mouse-CD8-PerCP-Cy5.5; anti-mouse-CD25-Alexa488; anti-mouse-FOXP3-APC. Percentage of lymphocytes based on expression of surface markers and intracellular transcription FOXP3 factor was analyzed using a flow cytometer BD FACSAria™ III cellsorter in the DIVA software.

As shown in **Table 2** somatic protein solution of L4 *H. polygyrus* (L4) in the concentration range: 1.25 $\mu$g, 0.625 $\mu$g, 0.312 $\mu$g, 0.156 $\mu$g, 0.078 $\mu$g and 0039 $\mu$g per 100,000 PBMC cells, significantly increases the percentage of CD8$^+$ lymphocytes, including CD8$^+$CD25$^+$ lymphocytes with the highest effectiveness for the antigen concentration of 0.078 $\mu$g. Whereas, the effectiveness of the antigen in CD8$^+$CD25$^+$ lymphocyte induction was greater than in CD4$^+$CD25$^+$ lymphocyte induction. As shown in **Table 3** which shows the results of the viability analysis and cellular apoptosis for PBMCs isolated from blood of the EAE mice from *in vitro* cultures treated with different concentrations of the somatic protein solution of L4 *H. polygyrus* larvae for 120h, the presence of the L4 stadium larval protein solution in PBMC blood culture does not significantly reduce the viability of the cells (less than 10% reduction in viability in the range up to 5 $\mu$g of protein / 100,000 cells in the culture).

**Table 2.** Percentage of lymphocytes with the CD8$^+$, CD8$^+$CD25$^+$ phenotype, including lymphocytes with the CD8$^+$CD25$^+$ phenotype with FOXP3 expression after 120h *in vitro* culture of PBMCs isolated from EAE mice in the presence of different concentrations of the somatic protein solution of L4 *H. polygyrus*.

| STIMULATION | Lymphocytes CD8+ (%) | Lymphocytes CD8+CD25+ (%) | Lymphocytes CD8+CD25+ with FOXP3 (%) | Lymphocytes CD4+ (%) | Lymphocytes CD4+CD25+ (%) | Lymphocytes CD4+CD25+ with FOXP3 (%) |
|---|---|---|---|---|---|---|
| 100,000 PBMC Lack of stimulation | 18.93±2.11 | 5.36±0.42 | 39.16±4.81 | 1.71±0.21 | 0.29±0.05 | 96.69±3.11 |
| L4 5 µg /100,000 PBMC | 18.03±3.18 | 5.39±1.10 | 38.6±8.91 | 1.64±0.3 | 0.30±0.01 | 96.82±4.86 |
| L4 2.5 µg | 17.62±2.10 | 5.89±1.15 | 30.79±3.62 | 1.45±0.25 | 0.32±0.06 | 98.52±4.82 |
| L4 1.25 µg | 42.86±3.92 | 12.84±2.31 | 32.50±4.12 | 3.31±0.14 | 0.48±0.02 | 98.14±5.48 |
| L4 0.625 µg | 47.09±4.71 | 16.62±2.11 | 30.86±2.34 | 3.55±0.17 | 0.66±0.03 | 94.81±4.16 |
| L4 0.312 µg | 55.56±5.30 | 17.79±1.98 | 35.90±2.98 | 4.37±0.34 | 0.78±0.05 | 99.30±4.13 |
| L4 0.156 µg | 56.98±4.98 | 18.94±1.99 | 34.20±2.65 | 4.04±0.21 | 0.54±0.02 | 98.30±3.54 |
| L4 0.078 µg | 55.69±4.99 | 22.45±2.67 | 33.30±2.34 | 4.03±0.19 | 0.48±0.02 | 97.6±3.40 |
| L4 0.039 µg | 56.87±5.01 | 18.8±2.02 | 34.35±3.12 | 4.15±0.09 | 0.45±0.02 | 98.9±4.29 |

**Table 3.** Results for cell viability and apoptosis of PBMCs isolated from blood of EAE mice from *in vitro* cultures treated with different concentrations of the somatic protein solution of L4 *H. polygyrus* larvae for 120h using Muse Annexin V & Dead Cell Kit in the cell analyzer Muse (Millipore). The mean cell viability in the culture is shown.

| STIMULATION | Living cells (%) | Apoptotic cells (%) |
|---|---|---|
| 100,000. PBMC Lack of stimulation | 92.0±4.05 | 8.0±1.02 |
| L4 5 µg | 83.3±4.20 | 16.7±2.89 |
| L4 2.5 µg | 90.95±3.87 | 9.05±1.08 |
| L4 1.25 µg | 93.1±4.56 | 6.90±1.05 |
| L4 0.625 µg | 93.15±5.76 | 6.85±0.91 |
| L4 0.312 µg | 94.75±4.67 | 6.25±0.89 |
| L4 0.156 µg | 93.90±3.87 | 6.10±0.67 |
| L4 0.078 µg | 93.85±3.58 | 6.15±0.78 |
| L4 0.039 µg | 93.17±3.47 | 6.83±0.87 |

**Example 6**

**Infection with the *H. polygyrus* nematode induces lymphocytes of the CD8+CD25+FOXP3 population without CD103 expression in mice with autoimmune encephalomyelitis, EAE**

Mice of the C57Bl6 stain, females at the age of 8 weeks, were actively immunized in order to induce EAE (experimental autoimmune encephalomyelitis) according to Example 1. Non-immunized control mice were administered PBS 7.2 buffer alone, in the same way.

The animals were observed every day and the aggravation of neurological symptoms of EAE was evaluated, as shown in Example 1. From the 19th day after immunization, aggravation of EAE symptoms was observed. On the 21st day after immunization, the animals were infected with 300 *H. polygyrus* larvae in the L3 stage, suspended in 200 µl of drinking water, using an esophageal probe. The animals which constituted the group of mice with EAE, not-infected with the nematode were administered 200 µl of drinking water in the same way. Aggravation of the clinical symptoms in animals was evaluated.

On the 6th day of infection, cerebrospinal fluid (CSF, at the volume of 20 µL) was collected from the animals using a capillary inserted between occipital nodules and top vertebra of the spine. CSF cells were phenotypically analyzed based on the surface markers using rat anti-mouse antibodies coupled with fluorescent dyes: anti-CD25 coupled with PE-Cy7; anti-CD8 coupled with APC; anti-CD4 coupled with PercP; anti-CD103 couple with z FITC, and the level of the intracellular FOXP3 transcription factor was identified using the anti-FOXP3 anti-PE antibody in the FACSVerse (BD) flow cytometer. The analysis was performed using FCS Express 5 De Novo Software. The experiment was repeated on groups of 5 animals.

On the 6th day after the infection with the nematode, the lack of clinical symptoms of EAE correlated with an increase in the percentage of lymphocytes with the CD8+CD25+ phenotype from the mean level 1.6 ±0.2% in mice with EAE to 6.0 ± 0.4% in mice with EAE infected with *H. polygyrus* as well as with a decrease in CD103+ lymphocytes among all the CD8+CD25+ lymphocytes from the mean level of 85.5 ±0.2% for mice with EAE to 51 ± 4.5% for mice with EAE infected with *H. polygyrus*. Infection of the mice with the nematode did not increase the percentage of CD103 positive cells in the CD8+CD25+FOXP3- population. These cells also did not show increased expression of CD103, calculated based on the MFI: median fluorescence intensity.

It follows that the infection with the *H. polygyrus* nematode induces the CD8$^+$CD25$^+$FOXP3$^-$CD103$^-$ lymphocyte population in mice with experimental autoimmune encephalomyelitis, that is in the mouse model of multiple sclerosis. Thus, it was shown that during the infection of EAE mice with *H. polygyrus,* the population of suppressor CD8$^+$CD25$^+$FOXP3$^-$CD103$^-$ lymphocytes is activated.

**Example 7**

**_Ex vivo_ induction of lymphocytes from blood of patients with MS.**

The study was performed on 3 patients with a progressive-relapsing MS who were not subject to any immunotherapy during blood donations.

The patients with MS were recruited from the patients of the MS Subdivision of the Neurology Department and Clinic of the Medical University of Warsaw.

Blood samples were collected to a volume of 10 ml by a vacuum method from an ulnar vein into Vacuette tubes with heparin sodium in the amount of 14 I.U./ml during routine testing so that the collection did not cause additional discomfort for the patient. Samples were stored and transported at room temperature. Peripheral blood mononuclear cells were isolated using Lymphoprep and placed in U-shaped wells of culture microplates at a density of 100,000 cells per well and cultured *ex vivo* in the presence of the solution of somatic protein of the L4 larvae of *H. polygyrus* at different concentrations of 5 $\mu$g; 2.5 $\mu$g; 1.25 $\mu$g; 0.625 $\mu$g; 0.312 $\mu$g; 0.156 $\mu$g; 0.078 $\mu$g; 0.039 $\mu$g in the total volume of the RPMI-1640 medium containing 10% of inactivated human serum (pooled normal human serum, NHS), 20 mM L-glutamine, 10 $\mu$g/ml gentamicin and 1M (HEPES). The solution of somatic proteins of the nematode was obtained according to

**Example 5.**

The cultures were carried out for 120 hours at the temperature of 37°C, in the atmosphere with 90% humidity, containing 95% of air and 5% of $CO_2$. After 24, 96, 120, 144 hours of culture cell viability was determined using Muse Annexin V & Dead Cell Kit in MUSE cell analyzer (Milipore) and after 120 hours of culture phenotypes were identified based on the surface markers using the following antibodies: anti-human-CD3-FITC; anti-human-CD8-PerCP, anti-human-CD25-PE-Cy7; anti-FOXP3-APC, anti-human-CD103-PE or anti-human-CD4-PE, and then, after permabilization, FOXP3 factor was labeled intracellularly using anti-FOXP3-APC antibodies. The percentage of the appropriate lymphocytes, among all the lymphocytes (expressing the CD3 surface marker) was calculated based on the expression of CD4, CD8, CD25, CD103 surface markers and the intracellular FOXP3 transcription factor by analysis using the flow cytometer BD FACSAria™ III cellsorter with the DIVA software. The median fluorescence intensity (MFI) was also determined for the CD25 surface marker, which allowed to distinguish the CD8$^+$CD25$^+$FOXP3$^-$CD103$^-$ populations with high (hi) and low (low) expression of the CD25 marker.

As it was shown in **Table 4,** the solution of somatic proteins of L4 *H. polygyrus* (L4) within the lowest concentration used, 0.039 $\mu$g per 100,000 PBMC cells significantly increases the percentage of CD8$^+$CD25$^+$FOXP3$^-$CD103$^-$ lymphocytes, including the lymphocytes with a high expression of CD25 (hi). As can be seen in **Table 5,** which shows the results of viability and apoptosis analysis of PBMC cells isolated from blood of the patients with MS in the *in vitro* cultures treated with different concentrations of the solution of somatic proteins of the L4 larva of *H. polygyrus* for 144 h, the presence of the solution of somatic proteins of the L4 stage larvae in the PBMC cells culture does not significantly decrease the viability of the cells to 120 h of culture (less than 10% reduction in viability in the applied range of protein concentrations/ 100,000 cells in the culture).

**Table 4** Percentage of lymphocytes with the CD3+CD8+ phenotype, including that with the expression of the surface markers CD25+ and CD103-, as well as the FOXP3- factor, including populations with low (low) and high expression (hi) of the CD25 marker without culturing (0 h) and after 120 h of culturing of PBMCs isolated from the blood of three patients with MS, in the presence of different concentrations of the solution of somatic proteins of L4 *H. polygyrus* (L4) and non-stimulated (MEDIUM). MFI-*Mean Fluorescence Intensity,* relates to the CD25 marker.

| Patient no 1 | CD3+ CD8+ | CD25+ FOXP3-CD103-in CD3+ CD8+ | CD25low FOXP3-CD103-in CD3+ CD8+ | CD25hi FOXP3-CD103-in CD3+ CD8+ | CD3+ CD4+ | CD25+ FOXP3-in CD3+CD4+ | CD25low FOXP3-in CD3+CD4+ | CD25hi FOXP3-in CD3+CD4+ |
|---|---|---|---|---|---|---|---|---|
| **0 h of culture** | 4.12 | 71.36 | 53.62 | 13.99 | 6.98 | 78.96 | 57.3 | 20.8 |
| **MEDIUM (%) MEDIUM (MFI)** | 404 | 70.12 4946.75 | 54.51 1842.78 | 10.61 14261.73 | 7.58 | 80.22 5223.75 | 58.44 1884.09 | 21.78 15243.62 |
| **L4 5ug (%) L4 5ug (MFI)** | 7.35 | 69.87 3937.8 | 58.81 1752.17 | 11.06 12681.5 | 9.97 | 78.03 8195 | 49.83 1784.71 | 28.2 18880.69 |
| **L4 2.5ug (%) L4 2.5ug (MFI)** | 9.53 | 67.16 3216.57 | 59.24 1627.36 | 7.92 12754.14 | 12.14 | 70.74 5145.38 | 53.26 1659.95 | 17.48 16575.52 |
| **L41.25ug (%) L4 1.25ug (MFI)** | 9.4 | 63.28 3272.6 | 53.99 1660.18 | 9.38 11332.9 | 11.97 | 68.01 4843.38 | 53.84 1683.56 | 14.17 17067.59 |
| **L4 0.625ug (%) L4 0.625ug (MFI)** | 9.9 | 73.37 3530.71 | 64.11 1770.87 | 9.26 14095.5 | 11.75 | 77.1 4952.66 | 62.19 1800.83 | 14.91 17649.78 |
| **L4 0.312ug (%) L4 0.312ug (MFI)** | 9.4 | 68.04 4198.28 | 58.25 1793.36 | 9.79 18630.78 | 13.21 | 74.18 4236.2 | 60.32 1812.39 | 13.86 17900.86 |
| **L4 0.156ug (%) L4 0.156ug (MFI)** | 8.27 | 72.9 3228.36 | 63.96 1875.76 | 9.3 11346.1 | 10.66 | 79.05 4956.37 | 60.89 1881.52 | 18.19 14362.96 |

EP 3 493 835 B1

| Patient no 1 | CD3+ CD8+ | CD25+ FOXP3-CD103-in CD3+ CD8+ | CD25low FOXP3-CD103-in CD3+ CD8+ | CD25hi FOXP3-CD103-in CD3+ CD8+ | CD3+ CD4+ | CD25+ FOXP3-in CD3+CD4+ | CD25low FOXP3-in CD3+CD4+ | CD25hi FOXP3-in CD3+CD4+ |
|---|---|---|---|---|---|---|---|---|
| L4 0.078ug (%) | 8.75 | 64.85 | 53.98 | 10.87 | 12.21 | 70.72 | 57.22 | 13.5 |
| L4 0.078ug (MFI) | | 5208.83 | 1806.28 | 22223.53 | | 4233.06 | 1848.98 | 18660.69 |
| L4 0.039ug (%) | 8.89 | 85.5 | 53.61 | 31.89 | 11.12 | 78.59 | 62.31 | 16.28 |
| L4 0.039ug (MFI) | | 4672 | 1941.76 | 11824.77 | | 4921.2 | 1953.13 | 17191.23 |

| Patient no. 2 | CD3+ CD8+ | CD25+ FOXP3-CD103- in CD3+ CD8+ | CD25low FOXP3-CD103- in CD3+ CD8+ | CD25hi FOXP3-CD103- in CD3+ CD8+ | CD3+ CD4+ | CD25+ FOXP3- in CD3+CD4+ | CD25low FOXP3-in CD3+CD4+ | CD25hi FOXP3-in CD3+CD4+ |
|---|---|---|---|---|---|---|---|---|
| 0 h of culture | 5.96 | 63.89 | 60.39 | 2.5 | 11.89 | 75.31 | 59.31 | 16.42 |
| MEDIUM (%) | 6.27 | 65.32 | 62.28 | 3.07 | 12.58 | 76.15 | 59.15 | 17 |
| MEDIUM (MFI) | | 2481.5 | 2005.11 | 12421 | | 4269.42 | 1724.47 | 17078.45 |
| L4 5ug (%) | 6.13 | 61.05 | 36.96 | 24.09 | 10.38 | 98.49 | 66.74 | 31.75 |
| L4 5ug (MFI) | | 7665.6 | 1776.89 | 16560.81 | | 6107 | 1359.16 | 17306.38 |
| L4 2.5ug (%) | 6.53 | 69.78 | 54.44 | 15.34 | 9.41 | 82.35 | 55.88 | 26.47 |
| L4 2.5ug (MFI) | | 4050.5 | 1936.39 | 14623.81 | | 5194 | 1886.24 | 14128.54 |
| L41.25ug (%) | 7.34 | 79.22 | 54.02 | 5.2 | 12.6 | 85.53 | 61.4 | 24.13 |
| L4 1.25ug (MFI) | | 2633.8 | 1935.48 | 12128.63 | | 4115.8 | 1818.52 | 11019.34 |
| L4 0.625ug (%) | 6.46 | 61.11 | 57.84 | 3.27 | 11.36 | 78.24 | 57.86 | 20.38 |
| L4 0.625ug (MFI) | | 3090.6 | 1894.3 | 13089.17 | | 5336.25 | 1778.63 | 16022.37 |

EP 3 493 835 B1

(continued)

| Patient no. 2 | CD3+ CD8+ | CD25+ FOXP3-CD103- in CD3+ CD8+ | CD25low FOXP3-CD103- in CD3+ CD8+ | CD25hi FOXP3-CD103- in CD3+ CD8+ | CD3+ CD4+ | CD25+ FOXP3- in CD3+CD4+ | CD25low FOXP3- in CD3+CD4+ | CD25hi FOXP3- in CD3+CD4+ |
|---|---|---|---|---|---|---|---|---|
| L4 0.312ug (%) | 7.01 | 61.54 | 56.63 | 4.91 | 13.33 | 78.75 | 59.11 | 19.64 |
| L4 0.312ug (MFI) | | 39144 | 2008.44 | 19432.39 | | 6474 | 1891 | 20451.38 |
| L4 0.156ug (%) | 6.46 | 65.65 | 62.57 | 308 | 13.19 | 77.53 | 62.29 | 15.24 |
| L40.166ug (MFI) | | 2395.5 | 1924.05 | 17221.17 | | 5468 | 1801.47 | 20208.24 |
| L4 0.078ug (%) | 503 | 64.51 | 61.46 | 3.05 | 12.88 | 12.88 | 62.82 | 16.12 |
| L4 0.078ug (MFI) | | 2520 | 1917.17 | 18139.45 | | 4981 | 1795.78 | 21590.72 |
| L4 0.039ug (%) | 5.59 | 79.51 | 62.42 | 17.09 | 16868.63 | 9.02 | 61.07 | 15.46 |
| L4 0.039ug (MFI) | | 3108.8 | 2149.81 | 16868.63 | | 5025 | 2078.68 | 17220.15 |

| Patient no. 3 | CD3+ CD8+ | CD25+ FOXP3-CD103- in CD3+ CD8+ | CD25low FOXP3-CD103- in CD3+ CD8+ | CD25hi FOXP3-CD103- in CD3+ CD8+ | CD3+ CD4+ | CD25+ FOXP3- in CD3+CD4+ | CD25low FOXP3- in CD3+CD4+ | CD25hi FOXP3- in CD3+CD4+ |
|---|---|---|---|---|---|---|---|---|
| 0 h of culture | 5.66 | 57.2 | 42.63 | 10.65 | 12.01 | 58.6 | 48.3 | 15.3 |
| MEDIUM (%) | 6.2 | 57.46 | 45.75 | 11.71 | 13.48 | 64.66 | 48.79 | 15.87 |
| MEDIUM (MFI) | | 4661.6 | 1928.31 | 15596.1 | | 5797.68 | 1956.82 | 25002 |
| L4 5ug (%) | 7.32 | 57.34 | 51.29 | 6.05 | 904 | 67.46 | 53 | 14.46 |
| L4 5ug (MFI) | | 3698.2 | 1842.53 | 16281.56 | | 6922.33 | 1860.2 | 32234.26 |
| L4 2.5ug (%) | 5.43 | 51.87 | 41.89 | 9.98 | 11.69 | 61.63 | 43.18 | 18.45 |
| L4 2.5ug (MFI) | | 4506.6 | 1747.18 | 15545.22 | | 8642.17 | 1723.77 | 22961.12 |

(continued)

| Patient no. 3 | CD3+ CD8+ | CD25+ FOXP3- CD103- in CD3+ CD8+ | CD25low FOXP3- CD103- in CD3+ CD8+ | CD25hi FOXP3- CD103- in CD3+ CD8+ | CD3+ CD4+ | CD25+ FOXP3- in CD3+CD4+ | CD25low FOXP3- in CD3+CD4+ | CD25hi FOXP3- in CD3+CD4+ |
|---|---|---|---|---|---|---|---|---|
| **L41.25ug (%)** <br> **L4 1.25ug (MFI)** | 6.15 | 57.92 <br> 4752.6 | 46.51 <br> 1836.92 | 11.41 <br> 15419.39 | 9.64 | 64.88 <br> 7557.42 | 46.41 <br> 1730.11 | 18.47 <br> 22151.83 |
| **L4 0.625ug (%)** <br> **L4 0.625ug (MFI)** | 5.74 | 53.06 <br> 4581.8 | 40.06 <br> 1861.68 | 13 <br> 14463.76 | 12.96 | 12.96 <br> 8488.33 | 42.1 <br> 1756.14 | 17.92 <br> 21953.74 |
| **L4 0.312ug (%)** <br> **L4 0.312ug (MFI)** | 5.96 | 54.94 <br> 4988.2 | 43.6 <br> 1809.23 | 11.34 <br> 16705.02 | 13.28 | 62.99 <br> 19502 | 42.6 <br> 1705.78 | 20.39 <br> 22701.14 |
| **L4 0.156ug (%)** <br> **L40.166ug (MFI)** | 6.86 | 66.95 <br> 2036.3 | 53.94 <br> 2024.34 | 13.01 <br> 1696.5 | 9.4 | 65.05 <br> 800985 | 47.73 <br> 1875.47 | 17.32 <br> 23694.93 |
| **L4 0.078ug (%)** <br> **L4 0.078ug (MFI)** | 6.98 | 64.62 <br> 6584.8 | 50.24 <br> 1915.93 | 14.38 <br> 20034.05 | 11.57 | 69.34 <br> 874768 | 50.05 <br> 1801.62 | 19.29 <br> 26112.39 |
| **L4 0.039ug (%)** <br> **L4 0.039ug (MFI)** | 7.48 | **75.29** <br> **5865.5** | **50.13** <br> **1979.28** | **25.16** <br> **17298.54** | 6.99 | 72.6 <br> 6023.42 | 49.67 <br> 2014.5 | 19.64 <br> 29547.3 |

**Table 5.** The percentage of live and apoptotic PBMC cells isolated from blood of patients with MS in *ex vivo* culture treated with different concentrations of the solution of somatic proteins of the L4 larvae of *H. polygyrus* (L4) for 0, 24, 96, 120 and 140 h using the Muse Annexin V & Dead Cell Kit in the Muse cell analyzer (Millipore). The viability of the cells of patient no. 1 in the culture is shown.

| Hour | MEDIUM | L4 5ug | L4 2.5ug | L4 1.25ug | L4 0.625ug | L4 0.312ug | L4 0.156ug | L4 0.078ug | L4 0.039ug |
|---|---|---|---|---|---|---|---|---|---|
| Live cells (%) | | | | | | | | | |
| 0 | 94.9 | | | | | | | | |
| 24 | 96 | 97.05 | 94.45 | 95.8 | 95.05 | 95.3 | 98.7 | 99.15 | 98.05 |
| 96 | 96.2 | 96.8 | 96.9 | 96.15 | 95.9 | 97.1 | 99.3 | 99.4 | 99.45 |
| 120 | 98.55 | 97.2 | 96.55 | 97.15 | 97.2 | 97.9 | 98.7 | 98.85 | 99.15 |
| 144 | 90.1 | 86.2 | 74.2 | 74.8 | 72.95 | 85.7 | 92.3 | 93.05 | 93.4 |
| Apoptotic cells (%) | | | | | | | | | |
| 0 | 4.1 | | | | | | | | |
| 24 | 3.1 | 2.7 | 3.75 | 3.6 | 3.7 | 3.2 | 0.9 | 0.8 | 1.7 |
| 96 | 0.6 | 3.2 | 3 | 3.8 | 4 | 2.9 | 0.65 | 0.5 | 0.45 |
| 120 | 0.1 | 2.5 | 3.3 | 2.6 | 2.7 | 1.9 | 1.2 | 1 | 0.85 |
| 144 | 1.95 | 5.55 | 6.8 | 6 | 7.45 | 4.3 | 2.8 | 1.1 | 1.15 |

**References**

Askenase P. W., Szczepanik M., Itakura A., Kiener C., Campos R. A.: Extravascular Tcell recruitment requires initiation begun by Valpha14+ NKT cells and B-1 B cells. Trends in Immunology 2004; 25: 441-449.

Donskow-Lysoniewska K., Krawczak K., Doligalska M. Heligmosomoides polygyrus: EAE remission is correlated with different systemic cytokine profiles provoked by L4 and adult nematodes. Experimental Parasitology 2012 132(2): 243-248.

Fleming J. O., J. O., Isaak A., Lee J. E., Luzzio C. C., Carrithers M. D., Cook T. D., & Fabr, Z. Probiotic helminth administration in relapsing-remitting multiple sclerosis: a phase 1 study. Multiple Sclerosis Journal 2011 17(6): 743-754.

Friese MA, Fugger L. Autoreactive CD8+ T cells in multiple sclerosis: a new 486 target for therapy? Brain 2005 128 (8):1747-63.

Gonzalez-Rey E., Fernandez-Martin A., Chorny A., & Delgado M. Vasoactive intestinal peptide induces CD4+, CD25+ T regulatory cells with therapeutic effect in collagen-induced arthritis. Arthritis & Rheumatism 2006 54(3): 864-876.

Gryglewski A, Majcher P, Szczepaniec M. Immunologiczne aspekty urazu. Postępy Higieny i Medycyny Doświad-czalnej 200660: 192-200.

Gudjonsson JE, Johnston A, Sigmundsdottir H, Valdimarsson H. Immunopathogenetic mechanisms in psoriasis. Clinical & Experimental Immunology 2004 135: 1-8.

Järnerot G., Rolny P., and Sandberg-Gertzen H. Intensive intravenous treatment of ulcerative colitis. Gastroenterology 1985 89(5): 1005-1013.

Johnston C. J., Robertson E., Harcus Y., Grainger, J. R., Coakley G., Smyth D. J., & Maizels, R.. Cultivation of Heligmosomoides polygyrus: an immunomodulatory nematode parasite and its secreted products. JoVE. Journal of Visualized Experiments 2005 98: e52412-e52412.

Kohm A. P., Carpentier P. A., Anger H. A., & Miller S. D. Cutting edge: CD4+ CD25+ regulatory T cells suppress antigen-specific autoreactive immune responses and central nervous system inflammation during active experimental autoimmune encephalomyelitis. The Journal of Immunology 2002 169 (9): 4712-4716.

Kułakowska A., Bartosik-Psujek H., Hozejowski R., Mitosek-Szewczyk K., Drozdowski W., Stelmasiak Z. Selected aspects of the epidemiology of multiple sclerosis in Poland - a multicenter pilot study. *Neurologia i Neurochirurgia Polska* 2010 44(5): 443-452.

Lakatos Peter Laszlo. Recent trends in the epidemiology of inflammatory bowel diseases: up or down? World Journal of Gastroenterology 2006 12(38): 6102-08.

Lewis C. A., Manning J., Rossi F., & Krieger C. The neuroinflammatory response in ALS: the roles of microglia and T cells. Neurology Research International 2012.

Liu Y., Xu A. P., Horwitz D. A., & Zheng, S. G. (2012). Cd8+ foxp3-cd103+ Regulatory T Cells Generated Ex Vivo with Tgf-β Suppress Autoimmunity Through Il-10-dependet Mechanism. Arthritis & Rheumatism, 64, S105.

Liu, Y., Lan, Q., Lu, L., Chen, M., Xia, Z., Ma, J., ... & Brand, D. (2013). Phenotypic and functional characteristic of a newly identified CD8+ Foxp3- CD103+ regulatory T cells. Journal of Molecular Cell Biology, 6(1), 81-92.

Lu Q.: The critical importance of epigenetics in autoimmunity. Journal of autoimmunity 2013 41: 1-5.

Maloy KJ, Powrie F. Regulatory T cells in the control of immune pathology. Nature Immunology 2001 2: 816-22.

A., Świątkowski M., Meder G., Koza J., & Szamocka M. Treatment costs for the group of patients with non-specific inflammatory bowel disease during acute exacerbation and further annual observation. *Przegląd Gastroenterologiczny* 2011 6(1): 36-44.

Matricardi P., Meo C. D., Coviello T., & Alhaique F. Recent advances and perspectives on coated alginate microspheres for modified drug delivery. Expert Opinion on Drug Delivery 2008 5(4): 417-425.

McSorley, H. J., Hewitson J. P., & Maizels R. M. (2013). Immunomodulation by helminth parasites: defining mechanisms and mediators. International journal for parasitology, 43(3), 301-310.

Molodecky N. A., Soon S., Rabi D. M., Ghali W. A., Ferris M., Chernoff G., & Kaplan, G. G. Increasing incidence and prevalence of the inflammatory bowel diseases with time, based on systematic review. Gastroenterology 2012 142(1): 46- 54.

MP, Medycyna Praktyczna - Indeks Leków 2014.

Palasik W. Leki biologiczne w leczeniu stwardnienia rozsianego. Przegląd aktualnych osiągnięć. Biological treatment in multiple sclerosis. Review current development. *Postępy Nauk Medycznych* 2013 10: 715-719.

Piskin G., Tursen U., Sylva-Steenland R. M. R., Bos J. D., & Teunissen M. B. M. Clinical improvement in chronic plaque-type psoriasis lesions after narrow-band UVB therapy is accompanied by a decrease in the expression of IFN-γ inducers-IL-12, IL-18 and IL-23. Experimental dermatology 2004 13(12): 764-772.

Potemkowski A. Stwardnienie rozsiane w swiecie i w Polsce - ocena neurologiczna. *Aktualnosci Neurologiczne* 2009 91-97.

Putzki N, Hartung HP: Treatment of multiple sclerosis. Uni-Med 2009 16-22.

Ruyssers N. E., De Winter B. Y., De Man J. G., Loukas A., Herman A. G., Pelckmans P. A., & Moreels T. G. Worms and the treatment of inflammatory bowel disease: are molecules the answer? Clinical and Developmental Immunology 2008 (1): 2.

Satsangi J., Silverberg M. S., Vermeire S., & Colombel J. The Montreal classification of inflammatory bowel disease: controversies, consensus, and implications. Gut 2006 55(6): 749-753.

Selmaj K: Leczenie przyczynowe stwardnienia rozsianego. *Polski Przegląd Neurologiczny* 2005 1(3):118-123.

Stock P., Kallinich T., Akbari O., Quarcoo D., Gerhold K., Wahn U., & Hamelmann 490 E. CD8+ T cells regulate immune responses in a murine model of allergen-491 induced sensitization and airway inflammation. European Journal of Immunology 2004 34(7): 1817-1827.

Valanparambil R. M., Segura M., Tam M., Jardim A., Geary T. G., & Stevenson M. M. Production and analysis of immunomodulatory excretory-secretory products from the mouse gastrointestinal nematode Heligmosomoides polygyrus bakeri. Nature Protocols 2014 9(12): 2740-2754.

## Claims

1. A method for ex *vivo* induction of lymphocytes with a CD8$^+$CD25$^+$ phenotype, **characterized in that** isolated blood cells of an individual, are ex *vivo* contacted with a solution of somatic proteins from a helminth parasite,

   wherein the solution of somatic proteins is obtained by collecting developmental stage of a helminth L4 larvae stage and shredding in order to obtain a complete tissue homogenate, and wherein helminth parasite is a nematode *Heligmosomoides polygyrus;*
   wherein the contacting is carried out under conditions and on a medium enabling proliferation of lymphocytes,

wherein the induced lymphocytes are then isolated,

wherein the obtained isolated lymphocytes with the CD8+CD25+ phenotype do not express the transcription factor FOXP3 and are CD103-;

wherein the L4 stage of development of *Heligmosomoides polygyrus* is obtained by infection of a non-human animal with an invasive form, wherein preferably the infection is with the L3 stage of development of *Heligmosomoides polygyrus;*

cultivation of the non-human animal for the time needed for the larva to transform into the next stage L4, in which it colonizes the small intestine;

isolation of the small intestine of the non-human animal and release of the developed L4 stage into the medium and its separation from the medium; and

wherein isolated blood cells of an individual are from individual exhibiting presence of autoreactive T lymphocytes.

2. The method for ex *vivo* induction of lymphocytes with a CD8+CD25+ phenotype, according to claim 1, **characterized in that** the isolated blood cells of an individual come from a human;

wherein preferably isolated blood cells are whole blood or peripheral blood mononuclear cells.

3. The method for *ex vivo* induction of lymphocytes with a CD8+CD25+ phenotype, according to claims 1-2, **characterized in that** the proliferation of the lymphocytes is carried out in a culturing medium enabling proliferation of lymphocytes containing 10% inactivated human serum, for 24-120 hours under the conditions of 37°C, 5%CO$_2$ and 95% humidity.

4. The method for *ex vivo* induction of lymphocytes with a CD8+CD25+ phenotype, according to claims 1-3, **characterized in that** the obtained induced lymphocytes with the phenotype CD8+CD25+ FOXP3-CD103-, are then isolated based on the expression of markers CD8, CD25, CD103, preferably also CD3, using a cell sorter, using appropriately labeled monoclonal antibodies against the particular markers, preferably anti-human antibodies, and the FOXP3- factor is also identified.

**Patentansprüche**

1. Verfahren zur ex *vivo* Induktion von Lymphozyten mit einem CD8+CD25+ Phänotyp, **dadurch gekennzeichnet, dass** isolierte Blutzellen eines Individuums ex *vivo* mit einer Lösung von somatischen Proteinen eines Helminthen-Parasiten in Kontakt gebracht werden,

wobei die Lösung somatischer Proteine durch Sammeln des Entwicklungsstadiums einer Helminthen-Larve im Stadium L4 und Zerkleinern erhalten wird, um ein vollständiges Gewebehomogenat zu erhalten, und wobei der Helminthen-Parasit ein Nematode *Heligmosomoides polygyrus* ist;

wobei die Kontaktierung unter Bedingungen und auf einem Medium durchgeführt wird, das die Proliferation von Lymphozyten ermöglicht,

wobei die induzierten Lymphozyten dann isoliert werden,

wobei die erhaltenen isolierten Lymphozyten mit dem CD8+CD25+ Phänotyp den Transkriptionsfaktor FOXP3 nicht exprimieren und CD103- sind;

wobei das L4-Stadium der Entwicklung von *Heligmosomoides polygyrus* durch Infektion eines nichtmenschlichen Tieres mit einer invasiven Form erhalten wird, wobei die Infektion vorzugsweise mit dem L3-Stadium der Entwicklung von *Heligmosomoides polygyrus* erfolgt:

Kultivierung des nichtmenschlichen Tieres für die Zeit, die die Larve benötigt, um sich in das nächste L4-Stadium zu verwandeln, in dem sie den Dünndarm besiedelt;

Isolierung des Dünndarms des nichtmenschlichen Tieres und Freisetzung des entwickelten L4-Stadiums in das Medium und seine Abtrennung vom Medium; und

Wobei die isolierten Blutzellen eines Individuums von einem Individuum stammen, das autoreaktive T-Lymphozyten aufweist.

2. Verfahren zur Ex-vivo-Induktion von Lymphozyten mit einem CD8+CD25+ Phänotyp, nach Anspruch 1, **dadurch gekennzeichnet, dass** die isolierten Blutzellen eines Individuums von einem Menschen stammen;

wobei es sich bei den isolierten Blutzellen vorzugsweise um Vollblut oder mononukleäre Zellen aus peripherem Blut handelt.

**3.** Verfahren zur Ex-vivo-Induktion von Lymphozyten mit einem CD8$^+$CD25$^+$ Phänotyp gemäß den Ansprüchen 1 bis 2, **dadurch gekennzeichnet, dass** die Proliferation der Lymphozyten in einem Kulturmedium, das die Proliferation von Lymphozyten ermöglicht und 10 % inaktiviertes Humanserum enthält, während 24 bis 120 Stunden unter den Bedingungen von 37°C, 5% CO$_2$ und 95% Feuchtigkeit durchgeführt wird.

**4.** Verfahren zur Ex-vivo-Induktion von Lymphozyten mit einem CD8$^+$CD25$^+$ Phänotyp, nach Ansprüchen 1 bis 3, **dadurch gekennzeichnet, dass** die erhaltenen induzierten Lymphozyten mit dem Phänotyp CD8$^+$CD25$^+$FOXP3CD103$^-$ anschließend auf der Grundlage der Expression der Marker CD8, CD25, CD103, vorzugsweise auch CD3, unter Verwendung eines Zellsortierers isoliert werden, wobei entsprechend markierte monoklonale Antikörper gegen die jeweiligen Marker, vorzugsweise Anti-Human-Antikörper, verwendet werden, und der Faktor FOXP3-ebenfalls identifiziert wird.

## Revendications

**1.** Un procédé d'induction ex vivo de lymphocytes de phénotype CD8$^+$CD25$^+$, **caractérisé en ce que** des cellules sanguines isolées d'un individu sont mises en contact ex vivo avec une solution de protéines somatiques d'un parasite helminthique,

> dans lequel la solution de protéines somatiques est obtenue en collectant le stade développemental d'un helminthe au stade de larve L4 et en le déchiquetant afin d'obtenir un homogénat de tissu complet, et dans lequel le parasite helminthique est un nématode Heligmosomoides polygyrus ;
> dans lequel la mise en contact est effectuée dans des conditions et sur un milieu qui permettent la prolifération des lymphocytes,
> dans lequel les lymphocytes induits sont ensuite isolés,
> dans lequel les lymphocytes isolés obtenus de phénotype CD8$^+$CD25$^+$ n'expriment pas le facteur de transcription FOXP3 et sont CD103- ;
> dans lequel le stade L4 du développement d'Heligmosomoides polygyrus est obtenu par l'infection d'un animal non humain par une forme invasive, l'infection étant de préférence au stade L3 du développement d'Heligmosomoides polygyrus :
>
>> la culture de l'animal non humain pendant le temps nécessaire à la transformation de la larve en stade L4, au cours duquel elle colonise l'intestin grêle ;
>> l'isolement de l'intestin grêle de l'animal non humain et la libération du stade L4 développé dans le milieu et sa séparation hors du milieu ; et
>> dans lequel les cellules sanguines isolées d'un individu proviennent d'un individu présentant des lymphocytes T autoréactifs.

**2.** Le procédé d'induction ex vivo de lymphocytes de phénotype CD8+CD25+ selon la revendication 1, **caractérisé en ce que** les cellules sanguines isolées d'un individu proviennent d'un humain ;
dans lequel les cellules sanguines isolées sont de préférence des cellules mononucléaires du sang total ou du sang périphérique.

**3.** Le procédé d'induction ex vivo de lymphocytes de phénotype CD8+CD25+, selon les revendications 1-2, **caractérisé en ce que** la prolifération des lymphocytes est réalisée dans un milieu de culture permettant la prolifération des lymphocytes contenant 10% de sérum humain inactivé, pendant 24 à 120 heures dans les conditions de 37°C, 5%COz et 95% d'humidité.

**4.** Le procédé d'induction ex vivo de lymphocytes de phénotype CD8$^+$CD25$^+$, selon les revendications 1-3, **caractérisé en ce que** les lymphocytes induits obtenus de phénotype CD8$^+$CD25$^+$FOXP3CD103$^-$, sont ensuite isolés sur la base de l'expression des marqueurs CD8, CD25, CD103, de préférence aussi CD3, à l'aide d'un trieur de cellules, en utilisant des anticorps monoclonaux marqués de manière appropriée contre les marqueurs particuliers, de préférence des anticorps anti-humains, et le facteur FOXP3- est également identifié.

## Fig. 1

# Fig. 2

**CSF of EAE mice**

CD25

2,7 ± 0,6%

5,0 ± 0,9%

**CSF of EAE mice infected with *H. polygyrus***

3,5 ± 0,6%

9,7 ± 1,1%*

CD4

CD8

**Fig. 3**

A

B

EAE Donor

EAE Donor infected with *H. polygyrus*

## Fig. 4

Mean Clinical Score of EAE symptoms

DONOR with EAE

DONOR with EAE
infected with
*H. polygyrus*

## Fig. 5

**Fig. 6**

## Fig. 7

**Fig. 8**

**Fig. 9**

# Fig. 10

**Fig. 11**

# Fig. 12

## FECAL CONSISTENCY

## HEMATOCRIT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012008133 A
- RU 2437933
- US 6521232 B1
- WO 2013039872 A1
- US 2005118655 A
- WO 2013192215 A1
- WO 2006081372 A2
- EP 1530972 A

### Non-patent literature cited in the description

- **LIU, Y. ; XU, A. P. ; HORWITZ, D. A. ; ZHENG, S. G.** Cd8+ foxp3-cd103+ Regulatory T Cells Generated Ex Vivo with Tgf-β Suppress Autoimmunity Through Il-10-dependent Mechanism. *Arthritis & Rheumatism,* 2012, vol. 64, S105
- **LIU, Y. ; LAN, Q. ; LU, L. ; CHEN, M. ; XIA, Z. ; MA, J. ; BRAND, D.** Phenotypic and functional characteristic of a newly identified CD8+ Foxp3- CD103+ regulatory T cells. *Journal of molecular cell biology,* 2013, vol. 6 (1), 81-92
- **ASKENASE P. W. ; SZCZEPANIK M. ; ITAKURA A. ; KIENER C. ; CAMPOS R. A.** Trends in Immunology. *Extravascular Tcell recruitment requires initiation begun by Valpha14+ NKT cells and B-1 B cells,* 2004, vol. 25, 441-449
- **DONSKOW-LYSONIEWSKA K. ; KRAWCZAK K. ; DOLIGALSKA M.** Heligmosomoides polygyrus: EAE remission is correlated with different systemic cytokine profiles provoked by L4 and adult nematodes. *Experimental Parasitology,* 2012, vol. 132 (2), 243-248
- **FLEMING J. O., J. O. ; ISAAK A. ; LEE J. E. ; LUZZIO C. C. ; CARRITHERS M. D. ; COOK T. D. ; FABR, Z.** Probiotic helminth administration in relapsing-remitting multiple sclerosis: a phase 1 study. *Multiple Sclerosis Journal,* 2011, vol. 17 (6), 743-754
- **FRIESE MA ; FUGGER L.** Autoreactive CD8+ T cells in multiple sclerosis: a new 486 target for therapy?. *Brain,* 2005, vol. 128 (8), 1747-63
- **GONZALEZ-REY E. ; FERNANDEZ-MARTIN A. ; CHORNY A. ; DELGADO M.** Vasoactive intestinal peptide induces CD4+, CD25+ T regulatory cells with therapeutic effect in collagen-induced arthritis. *Arthritis & Rheumatism,* 2006, vol. 54 (3), 864-876
- **GUDJONSSON JE ; JOHNSTON A ; SIGMUNDSDOTTIR H ; VALDIMARSSON H.** Immunopathogenetic mechanisms in psoriasis. *Clinical & Experimental Immunology,* 2004, vol. 135, 1-8
- **JÄRNEROT G. ; ROLNY P. ; SANDBERG-GERTZEN H.** Intensive intravenous treatment of ulcerative colitis. *Gastroenterology,* 1985, vol. 89 (5), 1005-1013
- **JOHNSTON C. J. ; ROBERTSON E. ; HARCUS Y. ; GRAINGER, J. R. ; COAKLEY G. ; SMYTH D. J. ; MAIZELS, R.** Cultivation of Heligmosomoides polygyrus: an immunomodulatory nematode parasite and its secreted products. *JoVE. Journal of Visualized Experiments,* 2005, vol. 98, e52412-e52412
- **KOHM A. P. ; CARPENTIER P. A. ; ANGER H. A. ; MILLER S. D.** Cutting edge: CD4+ CD25+ regulatory T cells suppress antigen-specific autoreactive immune responses and central nervous system inflammation during active experimental autoimmune encephalomyelitis. *The Journal of Immunology,* 2002, vol. 169 (9), 4712-4716
- **LAKATOS PETER LASZLO.** Recent trends in the epidemiology of inflammatory bowel diseases: up or down?. *World Journal of Gastroenterology,* 2006, vol. 12 (38), 6102-08
- **LEWIS C. A. ; MANNING J. ; ROSSI F. ; KRIEGER C.** The neuroinflammatory response in ALS: the roles of microglia and T cells. *Neurology Research International,* 2012
- **LIU Y. ; XU A. P. ; HORWITZ D. A. ; ZHENG,S. G.** Cd8+ foxp3-cd103+ Regulatory T Cells Generated Ex Vivo with Tgf-β Suppress Autoimmunity Through Il-10-dependet Mechanism. *Arthritis & Rheumatism,* 2012, vol. 64, S105
- **LIU, Y. ; LAN, Q. ; LU, L. ; CHEN, M. ; XIA, Z. ; MA, J. ; BRAND, D.** Phenotypic and functional characteristic of a newly identified CD8+ Foxp3- CD103+ regulatory T cells. *Journal of Molecular Cell Biology,* 2013, vol. 6 (1), 81-92
- **LU Q.** The critical importance of epigenetics in autoimmunity. *Journal of autoimmunity,* 2013, vol. 41, 1-5
- **MALOY KJ ; POWRIE F.** Regulatory T cells in the control of immune pathology. *Nature Immunology,* 2001, vol. 2, 816-22

- **MATRICARDI P. ; MEO C. D. ; COVIELLO T. ; AL-HAIQUE F.** Recent advances and perspectives on coated alginate microspheres for modified drug delivery. *Expert Opinion on Drug Delivery,* 2008, vol. 5 (4), 417-425
- **MCSORLEY, H. J. ; HEWITSON J. P ; MAIZELS R. M.** Immunomodulation by helminth parasites: defining mechanisms and mediators. *International journal for parasitology,* 2013, vol. 43 (3), 301-310
- **MOLODECKY N. A. ; SOON S. ; RABI D. M. ; GHALI W. A. ; FERRIS M. ; CHERNOFF G. ; KAPLAN, G. G.** Increasing incidence and prevalence of the inflammatory bowel diseases with time, based on systematic review. *Gastroenterology,* 2012, vol. 142 (1), 46-54
- **PISKIN G. ; TURSEN U. ; SYLVA-STEENLAND R. M. R. ; BOS J. D. ; TEUNISSEN M. B. M.** Clinical improvement in chronic plaque-type psoriasis lesions after narrow-band UVB therapy is accompanied by a decrease in the expression of IFN-γ inducers-IL-12, IL-18 and IL-23. *Experimental dermatology,* 2004, vol. 13 (12), 764-772
- **PUTZKI N ; HARTUNG HP.** Treatment of multiple sclerosis. *Uni-Med,* 2009, 16-22
- **RUYSSERS N. E. ; DE WINTER B. Y. ; DE MAN J. G. ; LOUKAS A. ; HERMAN A. G. ; PELCKMANS P. A. ; MOREELS T. G.** Worms and the treatment of inflammatory bowel disease: are molecules the answer?. *Clinical and Developmental Immunology,* 2008, vol. 1, 2
- **SATSANGI J. ; SILVERBERG M. S. ; VERMEIRE S. ; COLOMBEL J.** The Montreal classification of inflammatory bowel disease: controversies, consensus, and implications. *Gut,* 2006, vol. 55 (6), 749-753
- **STOCK P. ; KALLINICH T. ; AKBARI O. ; QUARCOO D. ; GERHOLD K. ; WAHN U. ; HAMELMANN.** 490 E. CD8+ T cells regulate immune responses in a murine model of allergen-491 induced sensitization and airway inflammation. *European Journal of Immunology,* 2004, vol. 34 (7), 1817-1827
- **VALANPARAMBIL R. M. ; SEGURA M. ; TAM M. ; JARDIM A. ; GEARY T. G. ; STEVENSON M. M.** Production and analysis of immunomodulatory excretory-secretory products from the mouse gastrointestinal nematode Heligmosomoides polygyrus bakeri. *Nature Protocols,* 2014, vol. 9 (12), 2740-2754